# EUROPÄISCHE PATENTSCHRIFT

(11) **EP 2 032 585 B1**
(45) Veröffentlichungstag und Bekanntmachung des Hinweises auf die Patenterteilung: **23.07.2014**
(21) Anmeldenummer: 07765267.5
(22) Anmeldetag: 15.05.2007
(51) Int. Cl.: C07D 519/00, C07D 475/08, A61K 31/519, A61K 31/55, A61P 35/00

(54) **SUBSTITUIERTE PTERIDINE ALS THERAPEUTIKA**
SUBSTITUTED PTERIDINES AS THERAPEUTIC AGENTS
PTERIDINES SUBSTITUTEÉS COMME AGENTS THERAPEUTIQUES

(30) Priorität: 24.05.2006 EP 06114538
(43) Veröffentlichungstag der Anmeldung: 11.03.2009
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: DOLLINGER, Horst, 88433 Schemmerhofen (DE); MARTYRES, Domnic, 88400 Biberach (DE); MACK, Juergen, 88400 Biberach (DE); GOEGGEL, Rolf, 89073 Ulm (DE); JUNG, Birgit, 88471 Laupheim (DE); NICKOLAUS, Peter, 88447 Warthausen (DE)
(74) Vertreter: Simon, Elke Anna Maria
(86) Internationale Anmeldenummer: PCT/EP2007/054703
(87) Internationale Veröffentlichungsnummer: WO 2007/135026

(56) Entgegenhaltungen:
- EP-A- 1 225 173
- WO-A-2004/056823
- WO-A-2006/056607
- WO-A-2006/058867
- DE-A1- 3 540 952

## Beschreibung

Die Erfindung betrifft neue Pteridine, die geeignet sind zur Behandlung von
- Atemwegs- oder gastrointestinalen Beschwerden oder Erkrankungen,
- entzündlichen Erkrankungen der Gelenke, der Haut oder der Augen,
- Erkrankungen des periphären oder zentralen Nervensystems oder
- Krebserkrankungen,
sowie pharmazeutische Zusammensetzungen die diese Verbindungen beinhalten.

### STAND DER TECHNIK

Pteridine sind als Wirkstoffe mit antiproliferativer Wirkung aus dem Stand der Technik bekannt. Merz et al. beschreiben im Journal of Medicinal Chemistry 1998, 41, 4733-4743 die Herstellung von 7-Benzylamino-6-chlor-2-piperazino-4 pyrrolidinopteridin und Derivaten davon, welche frei von Stellungsisomeren sind. Es wurde gezeigt, dass die hergestellten Verbindungen das Wachstum von Tumorzellen hemmen können. In der DE 3540952 werden 2-Piperazino-pteridine beschrieben, die in der 6-Stellung mit einem Halogenatom, ausgewählt aus einem Fluor-, Chlor- oder Bromatom, substituiert sind. Es wurde gezeigt, dass diese Verbindungen die Aktivität von Tumorzellen und von Humanthrombozyten in vitro hemmen konnten. Die DE 3323932 offenbart 2-Piperuzino-pteridine, die in der 4-Stellung eine Dialkylamino-, Piperidino-, Morpholino-, Thiomorpholino- oder 1-Oxidothiomorpholinogruppe tragen. Es wurde gezeigt, dass diese Verbindungen die Aktivität von Tumorzellen und von Humanthrombozyten in vitro hemmen konnten. In der DE 3445298 werden Pteridine mit einer großen Anzahl an unterschiedlichen Substituenten in der 2-, 4-, 6-und 7-Stellung beschrieben, wobei sich Verbindungen mit einer 2-Piperazinogruppe am Pteridingerüst als Hemmstoffe für das Tumorwachstum eignen sowie antithrombotische und metastasenhemmende Eigenschaften aufweisen. In der US 2,940,972 werden tri- und tetrasubstituierte Pteridinderivate offenbart, wobei allgemein Angaben gemacht werden, dass diese Pteridine wertvolle pharmakologische Eigenschaften, nämlich coronarerweiternde, sedative, antipyretische und analgetische Wirkungen aufweisen.

Die aus dem Stand der Technik bekannten Phosphodiesterase 4 Inhibitoren sind bekannt dafür, Nebenwirkungen wie Übelkeit und Erbrechen auszulösen (Doherty, 1999,Curr. Op. Chem. Biol., Aug. 3, (4):466-73). Die in dieser Erfindung benannten Substanzen hemmen bevorzugt die B-Isoenzyme der Phosphodiesterase 4, sind also bevorzugte PDE4B-Inhibitoren und sind infolgedessen besonders bevorzugt zur Behandlung der genannten Erkrankungen geeignet, da sie in einem Tiermodell für Übelkeit und Erbrechen (S. Murinus, Yamamoto K. et al.,Physiol. Behav., 2004, Oct. 30, 83(1), 151-6) diese Nebenwirkungen im Gegensatz zu anderen PDE4-Inhibitoren, die vorzugsweise die anderen PDE4-Isoenzyme (z.B. Isoenzyme A, C oderD) hemmen, nicht auslösen.

Aufgabe der vorliegenden Erfindung ist es neue Verbindungen bereit zu stellen, die geeignet sind zur Vorbeugung oder Behandlung von Atemwegs- oder gastrointestinalen Beschwerden oder Erkrankungen, entzündlichen Erkrankungen der Gelenke, der Haut oder der Augen, Erkrankungen des periphären oder zentralen Nervensystems, oder Krebserkrankungen, insbesondere solche Verbindungen, die durch geringere Nebenwirkungen, insbesondere Emesis und Nausea, gekennzeichnet sind.

### BESCHREIBUNG DER ERFINDUNG

Überraschenderweise konnte nun gefunden werden, dass Pteridine der Formel 1 geeignet sind zur Behandlung entzündlicher Erkrankungen.
Gegenstand der vorliegenden Erfindung sind deshalb Verbindungen der Formel 1 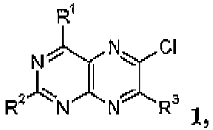 worin
- **R¹**: ein Rest ausgewählt aus der Gruppe bestehend aus einem gesättigten oder teilweise gesättigten vier-, fünf-, sechs- oder siebengliedrigen Heterocyclus, und ein fünf- oder sechsgliedriger Heteroaromat, der ein Stickstoffatom enthält und gegebenenfalls ein weiteres Atom ausgewählt aus der Gruppe bestehend aus Stickstoff, Schwefel und Sauerstoff enthalten kann;
und
- **R²**: ein Rest ausgewählt aus der Gruppe bestehend aus einem gesättigten oder teilweise gesättigten fünf-, sechs- oder siebengliedrigen Heterocyclus und einem fünf- oder sechsgliedrigen Heteroaromat, der ein Stickstoffatom enthält und gegebenenfalls ein weiteres Atom ausgewählt aus der Gruppe bestehend aus Stickstoff, Schwefel und Sauerstoff enthalten kann;
und worin
- **R³**: NR^{3.1}R^{3.2} oder OR^{3.1}, wobei
- **R**^{**3**.1} und **R^{3.2}**: jeweils unabhängig voneinander
H oder ein Rest ausgewählt aus der Gruppe bestehend aus C₁₋₆-Alkyl, ein- oder mehrwertiges, verzweigtes oder unverzweigtes C₁₋₆-Alkanol, C₁₋₆-Halogenalkyl, C₁₋₆-alkylen-O-C₁₋₂-Alkyl, ein mono- oder bicyclisches, gesättigtes oder teilweise gesättigtes C₃₋₁₀-Cycloalkyl-, mono- oder bicyclischer, gesättigter oder teilweise gesättigter, vier- bis zehngliedriger Heterocyclus mit 1 bis 3 Heteroatomen ausgewählt aus S, N oder O, und einem mono- oder bicyclischen, fünf- bis zehngliedrigen Heteroaromat mit 1 bis 4 Heteroatomen ausgewählt aus S, N oder O, der gegebenenfalls mit einem oder mehreren Resten ausgewählt aus der Gruppe bestehend aus OH, (Halogen), C₁₋₆-Alkyl, C₁₋₆-Alkanol, (C₁₋₆-Haloalkyl), COOR³³, O-C₁₋₆-Alkyl, C₆₋₁₀-Aryl, C₃₋₁₀-Cycloalkyl, vier- bis zehngliedriger Heterocyclus, fünfbis zehngliedriger Heteroaromat und O-C₁₋₄-Alkyl-Phenyl substituiert sein kann, wobei dieser Rest gegebenenfalls wiederum mit mindestens einem Rest ausgewählt aus der Gruppe bestehend aus Halogen, OH, C₁₋₃-Alkyl, C₁₋₃-Halogenalkyl substituiert sein kann,
und worin
- **R^{3.3}**: H, C₁₋₆-Alkyl oder (C₁₋₆-Alkanol),
oder worin
- **R³**: ein gesättigter oder teilweise gesättigter vier-, fünf-, sechs- oder siebengliedriger Heterocyclus, der ein Stickstoffatom enthält und gegebenenfalls ein oder zwei weitere Atome ausgewählt aus der Gruppe bestehend aus Stickstoff, Schwefel und Sauerstoff enthalten kann und
der gegebenenfalls mit einem oder mehreren Resten ausgewählt aus der Gruppe bestehend aus OH, Halogen, C₁₋₆-Alkyl, C₁₋₆-Alkanol, C₁₋₄-Alkyl-O-C₁₋₃-Alkyl, C₆₋₁₀-Aryl, C₃₋₁₀-Cycloalkyl, fünf- bis zehngliedriges Heteroaryl, vier- bis zehngliedriger Heterocyclus, der ein Stickstoffatom enthält und gegebenenfalls 1 oder 2 weitere Heteroatome ausgewählt aus N, S oder O enthalten kann, C₁₋₂-alkylen-C₅₋₁₀-Heteroaxyl und C₁₋₂-alkylen-C₄₋₁₀-Heterocyclus, der ein Stickstoffatom enthält und gegebenenfalls 1 oder 2 weitere Heteroatome ausgewählt aus N, S oder O enthalten kann, substituiert sein kann,
welcher wiederum gegebenenfalls durch einen oder mehrere Reste ausgewählt aus der Gruppe bestehend aus Methyl, Ethyl, O-Methyl, Cl, F und OH substituiert sein kann,
oder worin
- **R³**: ein gesättigter oder teilweise gesättigter, bi- oder polycyclischer sieben-, acht, neun- oder zehngliedriger Heterocyclus, der ein Stickstoffatom enthält und der gegebenenfalls ein, zwei oder drei weitere Atome ausgewählt aus der Gruppe bestehend aus Stickstoff, Schwefel und Sauerstoff enthalten kann und
der gegebenenfalls mit einem oder mehreren Resten ausgewählt aus der Gruppe bestehend aus OH, Halogen, C₁₋₆-Alkyl, C₁₋₆-Alkanol, C₁₋₄-Alkylen-O-C₁₋₃-Alkyl, C₆₋₁₀-Aryl, C₃₋₁₀-Cycloalkyl, fünf- bis zehngliedriges Heteroaryl, vier- bis zehngliedriger Heterocyclus, C₁₋₂-alkylen-C₅₋₁₀-Heteroaryl und C₁₋₂-alkylen-C₄₋₁₀-Heterocyclus substituiert sein kann,
welcher wiederum gegebenenfalls durch einen oder mehrere Reste ausgewählt aus der Gruppe bestehend aus Methyl, Ethyl, O-Methyl, Cl, F und OH substituiert sein kann,
bedeutet, sowie pharmakologisch verträgliche Salze, Diastereomere, Enantiomere, Racemate, Hydrate oder Solvate davon.

Besonders bevorzugt sind weiterhin Verbindungen der Formel 1, worin
- **R**¹: ein gesättigter oder ungesättigter vier-, fünf-oder sechsgliedriger Heterocyclus, oder Heteroaromat, der ein Stickstoffatom enthält und gegebenenfalls ein weiteres Atom ausgewählt aus der Gruppe bestehend aus Stickstoff, Schwefel und Sauerstoff enthalten kann;
und
- R²: ein fünf-, sechs- oder siebengliedriger Heterocyclus oder Heteroaromat, der ein Stickstoffatom enthält und gegebenenfalls ein weiteres Atom ausgewählt aus der Gruppe bestehend aus Stickstoff, Schwefel und Sauerstoff enthalten kann;
und worin
- **R³**: NR^{3.1}R^{3.2} oder OR^{3.1}, wobei
- **R**^{3.1} und **R**^{3.2}: jeweils unabhängig voneinander
H oder ein Rest ausgewählt aus der Gruppe bestehend aus C₁₋₆-Alkyl, ein- oder mehrwertiges, verzweigtes oder unverzweigtes C₁₋₆-Alkanol, C₁₋₆-Halogenalkyl, C₁₋₆-alkylen-O-C₁₋₂-Alkyl, mono- oder bicyclisches, gesättigtes oder teilweise gesättigtes C₃₋₁₀-Cycloalkyl, mono- oder bicyclischer, gesättigter oder teilweise gesättigter, vierbis zehngliedrigerHeterocyclus mit 1 oder 2 Heteroatomen ausgewählt aus S, N oder O, und einem mono- oder bicyclischen, fünf- bis zehngliedrigen Heteroaromat mit 1, 2 oder 3 Heteroatomen ausgewählt aus S, N oder O,
der gegebenenfalls mit einem oder mehreren Resten ausgewählt aus der Gruppe bestehend aus OH, C₁₋₆-Alkyl, C₁₋₆-Alkanol, COO-C₁₋₃-Alkyl, O-C₁₋₃-Alkyl, Phenyl, C₃₋₁₀-Cycloalkyl, vier- bis zehngliedriger Heterocyclus, fünf- bis zehngliedriger Heteroaromat und O-CH₂-Phenyl substituiert sein kann,
wobei dieser Rest gegebenenfalls wiederum mit mindestens einem Rest ausgewählt aus der Gruppe bestehend aus Halogen, OH, C₁₋₃-Alkyl, C₁₋₃-Halogenalkyl substituiert sein kann,
oder worin
- **R³**: ein gesättigter oder teilweise gesättigter vier-, fünf-, sechs- oder siebengliedriger Heterocyclus, der ein Stickstoffatom enthält und gegebenenfalls ein oder zwei weitere Atome ausgewählt aus der Gruppe bestehend aus Stickstoff, Schwefel und Sauerstoff enthalten kann und
der gegebenenfalls mit einem oder mehreren Resten ausgewählt aus der Gruppe bestehend aus OH, C₁₋₆-Alkyl, C₁₋₆-Alkanol, CH₂-O-CH₃, Phenyl, C₃₋₁₀-Cycloalkyl, fünf- bis zehngliedriges Heteroaryl, vier- bis zehngliedriger Heterocyclus, der ein Stickstoffatom enthält und gegebenenfalls 1 oder 2 weitere Heteroatome ausgewählt aus N, S oder O enthalten kann, CH₂-C₅₋₁₀-Heteroaryl und CH₂-C₄₋₁₀-Heterocyclus, der ein Stickstoffatom enthält und gegebenenfalls 1 oder 2 weitere Heteroatome ausgewählt aus N, S oder O enthalten kann, substituiert sein kann,
welcher wiederum gegebenenfalls durch einen oder mehrere Reste ausgewählt aus der Gruppe bestehend aus Methyl, O-Methyl, Cl und OH substituiert sein kann,
oder worin
- **R³**: ein gesättigter oder teilweise gesättigter, bi- oder polycyclischer sieben-, acht, neun- oder zehngliedriger Heterocyclus, der ein Stickstoffatom enthält und der gegebenenfalls ein, zwei oder drei weitere Atome ausgewählt aus der Gruppe bestehend aus Stickstoff, Schwefel und Sauerstoff enthalten kann und der gegebenenfalls mit einem oder mehreren Resten ausgewählt aus der Gruppe bestehend aus OH, C₁₋₆-Alkyl, C₁₋₆-Alkanol, CH₂-O-CH₃, Phenyl, C₃₋₁₀-Cycloalkyl, fünf- bis zehngliedriges Heteroaryl, vier- bis zehngliedriger Heterocyclus, CH₂-C₅₋₁₀-Heteroaryl und CH₂-C₄₋₁₀-Heterocyclus substituiert sein kann,
welcher wiederum gegebenenfalls durch einen oder mehrere Reste ausgewählt aus der Gruppe bestehend aus Methyl, O-Methyl, Cl und OH substituiert sein kann,
bedeutet, sowie pharmakologisch verträgliche Salze, Diastereomere, Enantiomere, Racemate, Hydrate oder Solvate davon.

Besonders bevorzugt sind weiterhin Verbindungen der Formel **1**, worin
R¹ und R² die oben genannten Bedeutungen haben
und worin
- **R³**: ein gesättigter oder teilweise gesättigter vier-, fünf-, sechs- oder siebengliedriger Heterocyclus, der ein Stickstoffatom enthält und über dieses Stickstoffatom mit dem Rest des Moleküls verknüpft ist und der gegebenenfalls ein oder zwei weitere Atome ausgewählt aus der Gruppe bestehend aus Stickstoff, Schwefel und Sauerstoff enthalten kann und
der gegebenenfalls mit einem oder mehreren Resten ausgewählt aus der Gruppe bestehend aus OH, (Halogen), C₁₋₆-Alkyl, C₁₋₆-Alkanol, C₁₋₄-Alkyl-O-C₁₋₃-Alkyl, C₆₋₁₀-Aryl, C₃₋₁₀-Cycloalkyl, fünf- bis zehngliedriges Heteroaryl, vier- bis zehngliedriger Heterocyclus, der ein Stickstoffatom enthält und gegebenenfalls 1 oder 2 weitere Heteroatome ausgewählt aus N, S oder O enthalten kann, C₁₋₂-alkylen-C₅₋₁₀-Heteroaryl und C₁₋₂-alkylen-C₄₋₁₀-Heterocyclus, der ein Stickstoffatom enthält und gegebenenfalls 1 oder 2 weitere Heteroatome ausgewählt aus N, S oder O enthalten kann, substituiert sein kann,
welcher wiederum gegebenenfalls durch einen oder mehrere Reste ausgewählt aus der Gruppe bestehend aus Methyl, Ethyl, O-Methyl, Cl, F und OH substituiert sein kann,
oder worin
- **R³**: ein gesättigter oder teilweise gesättigter, bi- oder polycyclischer sieben-, acht, neun- oder zehngliedriger Heterocyclus, der ein Stickstoffatom enthält und über dieses Stickstoffatom mit dem Rest des Moleküls verknüpft ist und der gegebenenfalls ein, zwei oder drei weitere Atome ausgewählt aus der Gruppe bestehend aus Stickstoff, Schwefel und Sauerstoff enthalten kann und der gegebenenfalls mit einem oder mehreren Resten ausgewählt aus der Gruppe bestehend aus OH, (Halogen), C₁₋₆-Alkyl, C₁₋₆-Alkanol, C₁₋₄-Alkyl-O-C₁₋₃-Alkyl, C₆₋₁₀-Aryl, C₃₋₁₀-Cycloalkyl, fünf- bis zehngliedriges Heteroaryl, vier- bis zehngliedriger Heterocyclus, C₁₋₂-alkylen-C₅₋₁₀-Heteroaryl und C₁₋₂-alkylen-C₄₋₁₀-Heterocyclus substituiert sein kann,
welcher wiederum gegebenenfalls durch einen oder mehrere Reste ausgewählt aus der Gruppe bestehend aus Methyl, Ethyl, O-Methyl, Cl, F und OH substituiert sein kann,
bedeutet,
sowie pharmakologisch verträgliche Salze, Diastereomere, Enantiomere, Racemate, Hydrate oder Solvate davon.

Besonders bevorzugt sind weiterhin Verbindungen der Formel 1, worin
- **R¹**: Pyrrolidin oder Azetidin bedeutet, sowie pharmakologisch verträgliche Salze, Diastereomere, Enantiomere, Racemate, Hydrate oder Solvate davon.

Besonders bevorzugt sind weiterhin Verbindungen der Formel **1**, worin
R¹ und R³ sowie R^{3.1}, R^{3.2} und R^{3.3} die oben genannten Bedeutungen haben
und worin
- **R²**: Piperazin bedeutet, sowie pharmakologisch verträgliche Salze, Diastereomere, Enantiomere, Racemate, Hydrate oder Solvate davon.

Besonders bevorzugt sind weiterhin Verbindungen der Formel 1, worin
R¹, R² und R^{3.1} die oben genannten Bedeutungen haben
und worin
- **R³**: NHR^{3.1} bedeutet,
sowie pharmakologisch verträgliche Salze, Diastereomere, Enantiomere, Racemate, Hydrate oder Solvate davon.

Besonders bevorzugt sind weiterhin Verbindungen der Formel **1**, worin
R¹ und R² die oben genannten Bedeutungen haben
worin
- **R³**: NHR^{3.2},
und worin
- **R^{3.2}**: ein verzweigtes oder unverzweigtes, ein- oder mehrwertiges C₁₋₆-Alkanol oder ein C₃₋₆-Cycloalkyl bedeutet, sowie pharmakologisch verträgliche Salze, Diastereomere, Enantiomere, Racemate, Hydrate oder Solvate davon.

Besonders bevorzugt sind weiterhin Verbindungen der Formel **1**, worin
- **R³**: NHR^{3.1} oder OR^{3.1}
ein gesättigter oder ungesättigter, fünf- oder sechsgliedriger Heterocyclus mit 1 oder 2 Heteroatomen unabhängig voneinander ausgewählt aus der Gruppe bestehend aus O, S und N,
- **R^{3.1}**: der gegebenenfalls mit einem der Reste aus der Gruppe
OH, Methyl, Ethyl, einem verzweigten oder unverzweigten C₁₋₄-Alkanol, Phenyl, C₃₋₁₀-Cycloalkyl, substituiert sein kann.

Besonders bevorzugt sind weiterhin die obigen Verbindungen der Formel **1**, worin
- **R³**: NHR^{3.1} oder OR^{3.1} und
- **R^{3.1}**: Tetrahydrofuryl oder Tetrahydropyranyl ist,
welcher gegebenenfalls mit einem der Reste aus der Gruppe
OH, Methyl, Ethyl, einem verzweigten oder unverzweigten C₁₋₄-Alkanol, Phenyl, C₃₋₁₀-Cycloalkyl, substituiert sein kann.

Besonders bevorzugt sind weiterhin Verbindungen der Formel 1, worin R¹ und R² die oben genannten Bedeutungen haben,
und worin
- **R³**: ein Rest ausgewählt aus der Gruppe bestehend aus einem gesättigten fünf-oder sechsgliedrigen Heterocyclus und einem bicyclischen gesättigten oder teilweise gesättigten acht-, neun- oder zehngliedrigen Heterocyclus, der ein Stickstoffatom enthält und über dieses Stickstoffatom mit dem Rest des Moleküls verknüpft ist und der gegebenenfalls ein weiteres Atom ausgewählt aus der Gruppe bestehend aus Stickstoff, Schwefel und Sauerstoff enthalten kann und der gegebenenfalls mit einem oder mehreren Resten ausgewählt aus der Gruppe bestehend aus OH, Methyl, Ethyl, einem verzweigten oder unverzweigten C₁₋₄-Alkanol, Phenyl, C₃₋₁₀-Cycloalkyl, einem fünf- bis zehngliedrigen Heteroaryl und einem vier- bis zehngliedrigen Heterocyclus substituiert sein kann, bedeutet,
sowie pharmakologisch verträgliche Salze, Diastereomere, Enantiomere, Racemate, Hydrate oder Solvate davon.

Besonders bevorzugt sind weiterhin Verbindungen der Formel 1, worin R¹ und R² die oben genannten Bedeutungen haben,
und worin
- **R³**: Pyrrolidin, das über das Stickstoffatom mit dem restlichen Molekül verknüpft ist und das gegebenenfalls mit einem oder mehreren Resten ausgewählt aus der Gruppe bestehend aus aus OH, Methyl, Ethyl und einem verzweigten oder unverzweigten C₁₋₄-Alkanol substituiert sein kann,
bedeutet,
sowie pharmakologisch verträgliche Salze, Diastereomere, Enantiomere, Racemate, Hydrate oder Solvate davon.

Besonders bevorzugt sind weiterhin Verbindungen der Formel **1**, worin
- **R¹**: 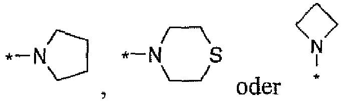
und
- **R²**: 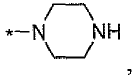
und
- **R³**: 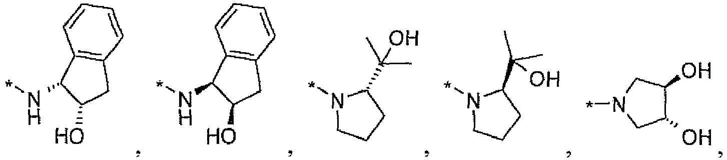 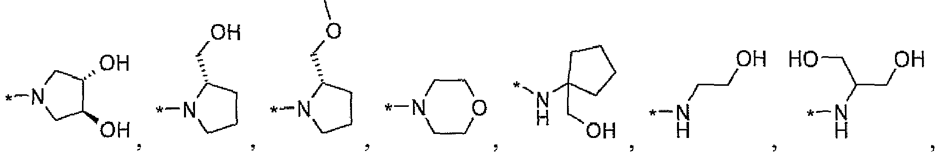 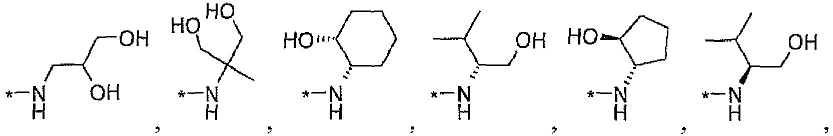 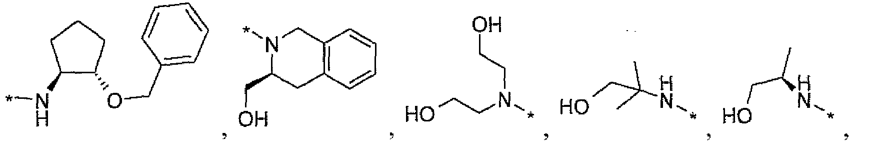 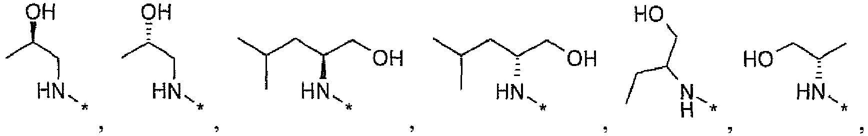 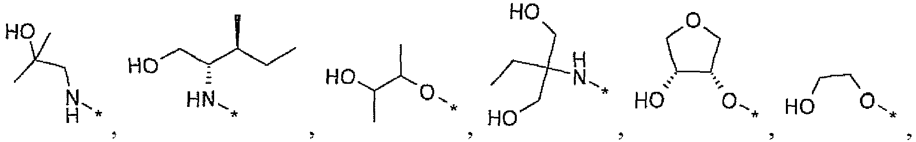 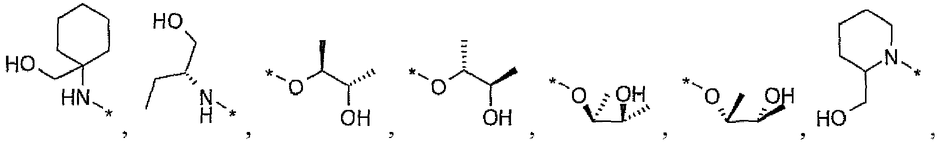 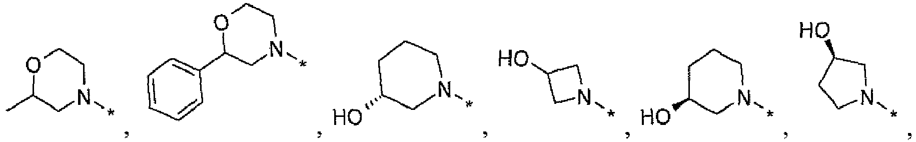 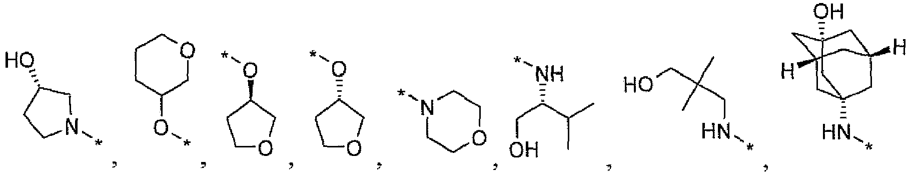 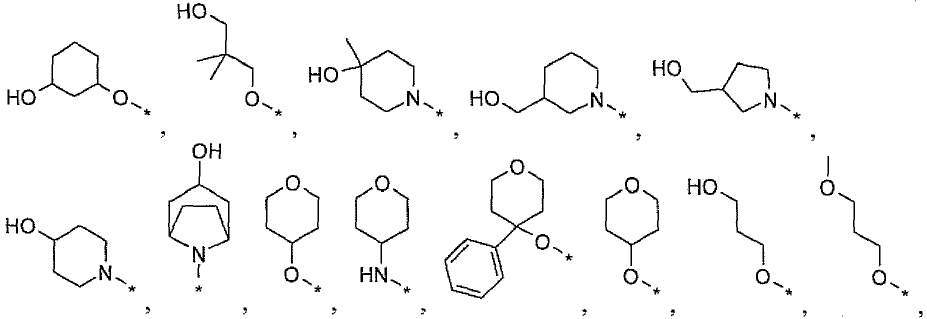 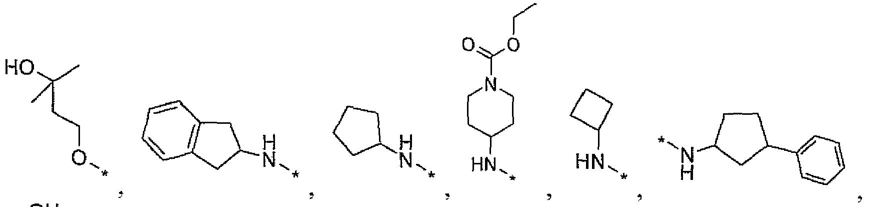 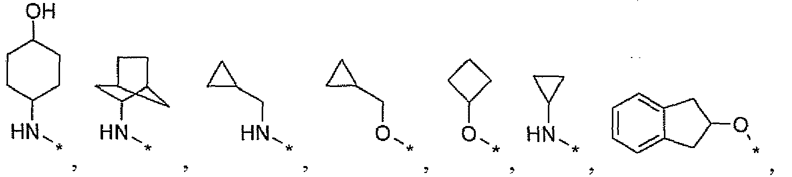 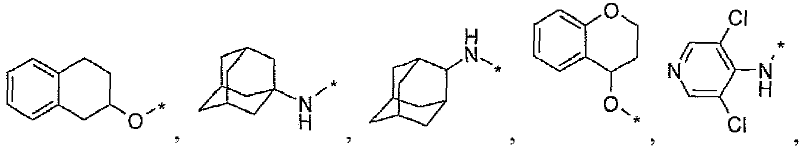 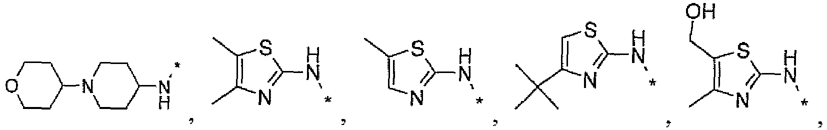 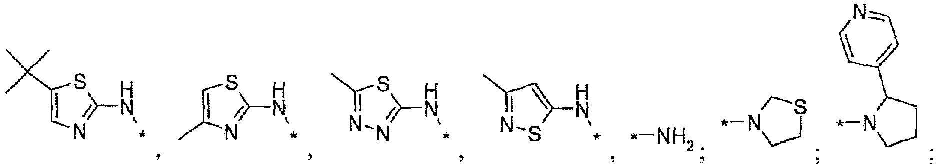 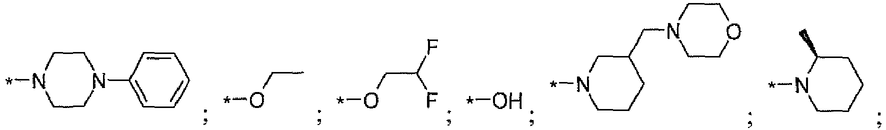 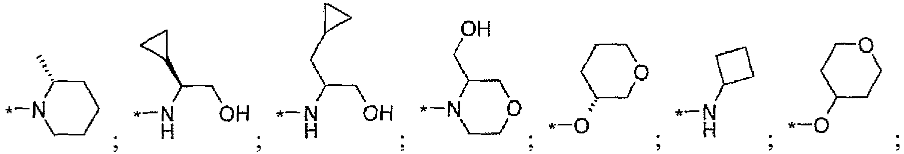 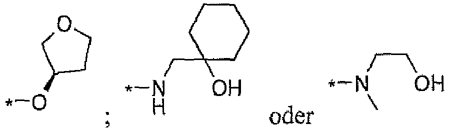
bedeutet, sowie pharmakologisch verträgliche Salze, Diastereomere, Enantiomere, Racemate, Hydrate oder Solvate davon.

Besonders bevorzugt sind weiterhin Verbindungen der Formel 2, 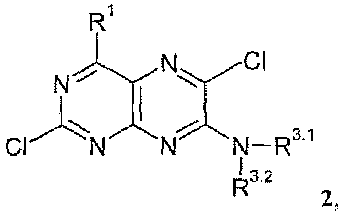 der Formel 3 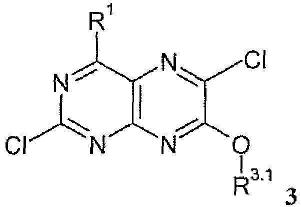 oder der Formel 4 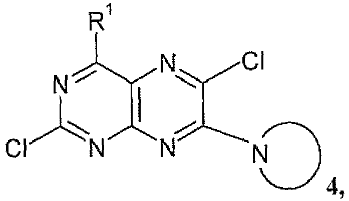 wobei R^{3.1} und R^{3.2} die oben definierten Bedeutungen haben,
wobei R¹ die oben definierten Bedeutungen haben, bevorzugt die die Bedeutungen Pyrrolidinyl, Azetidinyl oder Thiomorpholinyl haben,
und wobei 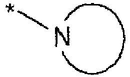 ausgewählt ist aus der Gruppe bestehend aus einem gesättigten oder teilweise gesättigten vier, fünf-, sechs- oder siebengliedrigen monocyclischen Heterocyclus oder einem sieben- bis zehngliedrigen bicyclischen Heterocyclus, der über ein Stickstoffatom mit dem Rest des Moleküls verknüpft ist und dergegebenenfalls ein oder zwei weitere Atome ausgewählt aus der Gruppe bestehend aus Stickstoff, Schwefel und Sauerstoff enthalten kann und
der gegebenenfalls mit einem oder mehreren Resten ausgewählt aus der Gruppe bestehend aus OH, Halogen, C₁₋₆-Alkyl, C₁₋₆-Alkanol, C₁₋₄-Alkyl-O-C₁₋₃-Alkyl, C₆₋₁₀-Aryl, C₃₋₁₀-Cycloalkyl, fünf- bis zehngliedriges Heteroaryl, vier- bis zehngliedriger Heterocyclus, C₁₋₂-alkylen-C₅₋₁₀-Heteroaryl und C₁₋₂-alkylen-C₄₋₁₀-Heterocyclus substituiert sein kann,
welcher wiederum gegebenenfalls durch einen oder mehrere Reste ausgewählt aus der Gruppe bestehend aus Methyl, Ethyl, O-Methyl, Cl, F und OH substituiert sein kann, sowie pharmakologisch verträgliche Salze, Diastereomere, Enantiomere, Racemate, Hydrate oder Solvate davon. Diese Verbindungen der Formeln 2, 3 und 4 stellen Zwischenprodukte der Herstellverfahren der Verbindungen der Formel 1 dar, die entsprechend der Reaktionsschemata 1, 2 oder 3 hergestellt werden.

Gegenstand der Erfindung sind weiterhin die oben genannten Verbindungen der Formel **1** zur Verwendung als Arzneimittel.

Ein weiterer Gegenstand betrifft die Verwendung der oben genannten Verbindungen , zur Herstellung eines Medikaments zur Behandlung von Krankheiten, die sich durch Inhibition des PDE4-Enzyms behandeln lassen.

Bevorzugt ist die Verwendung der oben genannten Verbindungen zur Herstellung eines Medikaments zur Behandlung von Atemwegserkrankungen, gastrointestinalen Beschwerden, entzündlichen Erkrankungen der Gelenke, der Haut oder der Augen, Krebserkrankungen und von Erkrankungen des periphären oder zentralen Nervensystems.

Besonders bevorzugt ist die Verwendung der oben genannten Verbindungen zur Herstellung eines Medikaments zur Vorbeugung und/oder zur Behandlung von Atemwegs- oder Lungenerkrankungen, die mit einer erhöhten Schleimproduktion, Entzündungen und / oder obstruktiven Erkrankungen der Atemwege einhergehen.

Ebenfalls bevorzugt ist die Verwendung der oben definierten Verbindungen zur Herstellung eines Medikaments zur Behandlung von entzündlichen Erkrankungen des Gastrointestinaltraktes.

Insbesondere betrifft die Erfindung die Verwendung der oben definierten Verbindungen zur Herstellung eines Medikament zur Behandlung von entzündlichen und/oder obstruktiven Erkrankungen wie COPD, chronischer Sinusitis, Asthma, Morbus Crohn, Colitis ulcerosa.

Weiterhin bevorzugt ist die Verwendung der oben definierten Verbindungen zur Herstellung eines Medikaments zur Vorbeugung und/oder Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Ebenfalls bevorzugt ist die Verwendung der oben definierten Verbindungen zur Herstellung eines Medikaments zur Behandlung von Krebserkrankungen wie z.B. akute und chronische Leukämien, akute lymphatischen Leukämie (ALL) und akute myeloische Leukämie (AML), chronisch lymphatische Leukämie (CLL) und chronisch myeloische Leukämie (CML), akute nicht lymphozytische Leukämie (ANLL), Haar-Zell-Leukämie, akute promyelocytische Leukämie (APL), insbesondere die APL-Subform mit einer chromosomalen t(15; 17)-Translokation, Erkrankungen der lymphatischen Organe, Hodgkin-Lymphome und non-Hodgkin Lymphome sowie von Knochentumoren wie z.B. das Osteosarkom und sowie alle Arten von Gliomen wie z.B. Oligodendrogliom und Glioblastom.

Bei den obigen Verwendungen der erfindungsgemäßen Pteridin-Verbindungen zur Herstellung eines Medikaments zur Vorbeugung und/oder Behandlung der oben genannten Erkrankungen sind in der Regel die Nebenwirkungen der Behandlung reduziert im Vergleich zu bekannten Therapeutika nach dem Stand der Technik.
Insbesondere sind die häufig auftretenden unerwünschten Nebenwirkungen Emesis und Nausea bei der Verwendung der oben definierten Verbindungen nach Formel **1** reduziert.

### VERWENDETE BEGRIFFE UND DEFINITIONEN

Im Rahmen dieser Anmeldung können bei der Definition von möglichen Substituenten, diese auch in Form einer Strukturformel dargestellt werden. Dabei wird ein Stern (*) in der Strukturformel des Substituenten als der Verknüpfungspunkt zum Rest des Moleküls verstanden. So werden zum Beispiel die Reste N-Piperidinyl (**I**), 4-Piperidinyl **(II),** 2-Tolyl **(III),** 3-Tolyl (**IV**) und 4-Tolyl **(V)** wie folgt dargestellt:

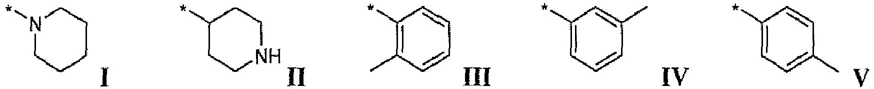

Befindet sich in der Strukturformel des Substituenten kein Stern (*), so kann an dem Substituenten jedes Wasserstoffatom entfernt werden und die dadurch freiwerdende Valenz als Bindungsstelle zum Rest eines Molekül dienen. So kann zum Beispiel **VI** die Bedeutung von 2-Tolyl, 3-Tolyl, 4-Tolyl und Benzyl haben.

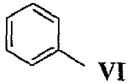

Unter pharmakologisch verträglichen Säureadditionssalzen werden beispielsweise diejenigen Salze verstanden, die ausgewählt sind aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-*p*-toluolsulfonat, bevorzugt Hydrochlorid, Hydrobromid, Hydrosulfat, Hydrophosphat, Hydrofumarat und Hydromethansulfonat.

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen verstanden und unter dem Begriff "C₁₋₄-Alkyl" verzweigte und unverzweigte Alkylgruppen mit 1 bis 4 Kohlenstoffatomen verstanden. Bevorzugt sind Alkylgruppen mit 1 bis 4 Kohlenstoffatome. Beispielsweise werden hierfür genannt: Methyl, Ethyl, *n*-Propyl, *iso*-Propyl, *n*-Butyl, *iso-*Butyl, *sec*-Butyl, *tert*-Butyl, *n*-Pentyl, *iso*-Pentyl, *neo*-Pentyl oder Hexyl. Gegebenenfalls werden für vorstehend genannten Gruppen auch die Abkürzungen Me, Et, n-Pr, *i*-Pr, *n*-Bu, i-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert-*Butyl etc.

Unter dem Begriff "C₁₋₆-Alkanol" (auch soweit sie Bestandteil anderer Reste sind) werden verzweigte und unverzweigte Alkylgruppen mit 1 bis 6 Kohlenstoffatomen, die mit einem oder mehreren Hydroxylgruppen substituiert sind, verstanden und unter dem Begriff "C₁₋₄-Alkanol" werden verzweigte und unverzweigte Alkylgruppen mit 1 bis 4 Kohlenstoffatomen verstanden, die mit einer oder mehreren Hydroxylgruppen substituiert sind. C₁₋₆-Alkanole, die mit einem Hydroxylgruppen substituiert sind, werden auch als "einwertige" C₁₋₆-Alkanole bezeichnet. C₁₋₆-Alkanole, die mit zwei oder mehreren Hydroxylgruppen substituiert sind, werden auch als "mehrwertige" C₁₋₆-Alkanole bezeichnet. Bevorzugt sind Alkanolgruppen mit 1 bis 4 Kohlenstoffatome. Beispielsweise werden hierfür genannt: CH₂-OH, Ethyl-OH, *n*-Propyl-OH, *n*-Butyl-OH, *iso*-Propyl-OH, *n*-Butyl-OH, *iso-*Butyl-OH, *sec*-Butyl-OH, *tert*-Butyl-OH, *n*-Pentyl-OH, *iso*-Pentyl-OH, *neo*-Pentyl-OH, Hexyl-OH,

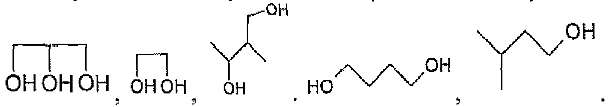

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatome. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc.

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Bevorzugt sind Alkinylgruppen mit 2 bis 4 Kohlenstoffatome. Beispielsweise werden hierfür genannt: Ethinyl, Propinyl, Butinyl, Pentinyl oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc.

Unter dem Begriff "C₁₋₄-Alkylen" bzw. "C₁₋₆-Alkylen (auch soweit sie Bestandteil anderer Reste sind) werden verzweigte und unverzweigte Alkylengruppen mit 1 bis 4 bzw. mit 1 bis 6 Kohlenstoffatomen verstanden. Beispielsweise werden hierfür genannt: Methylen, Ethylen, Propylen, 1-Methylethylen, Butylen, 1-Methylpropylen, 1,1-Dimethylethylen oder 1,2-Dimethylethylen. Sofern nicht anders beschrieben, umfassen die Definitionen Propylen und Butylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1,1-Dimethylethylen, 1,2-Dimethylethylen. Sollte die Kohlenstoffkette mit einem Rest substituiert sein, der gemeinsam mit einem oder zwei Kohlenstoffatomen der Alkylenkette einen carbocylischen Ring mit 3, 4, 5 oder 6 Kohlenstoffatomen bildet, so sind damit folgende Beispiele der Ringe umfasst:

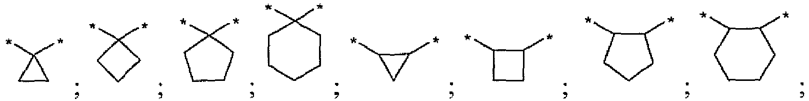

Unter dem Begriff "C₃₋₆-Cycloalkyl" (auch soweit sie Bestandteil anderer Reste sind) werden cyclische Alkylgruppen mit 3 bis 6 Kohlenstoffatomen verstanden. Beispielsweise werden hierfür genannt: Cyclopropyl, Cyclobutyl, Cyclopentyl oder Cyclohexyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff" C₇₋₁₁-Aralkyl" (auch soweit sie Bestandteil anderer Reste sind) werden verzweigte und unverzweigte Alkylgruppen mit 1 bis 5 Kohlenstoffatomen verstanden, die mit einem aromatische Ringsystem mit 6 Kohlenstoffatomen substituiert sind. Beispielsweise werden hierfür genannt: Benzyl, 1- oder 2-Phenylethyl. Soweit nicht anders beschreiben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Halogen steht im Rahmen der vorliegenden Erfindung für Fluor, Chlor, Brom oder Jod. Sofern nicht gegenteilig angegeben, gelten Fluor, Chlor und Brom als bevorzugte Halogene.

Unter dem Begriff "C₁₋₆-Haloalkyll" (auch soweit sie Bestandteil anderer Reste sind) werden verzweigte und unverzweigte Alkylgruppen mit 1 bis 6 Kohlenstoffatomen verstanden, die mit einem oder mehreren Halogenatomen substituiert sind. Unter dem Begriff "C₁₋₄-Haloalkyl" werden verzweigte und unverzweigte Alkylgruppen mit 1 bis 4 Kohlenstoffatomen verstanden, die mit einem oder mehreren Halogenatomen substituiert sind. Bevorzugt sind Alkylgruppen mit 1 bis 4 Kohlenstoffatome. Beispielsweise werden hierfür genannt: CF₃, CHF₂, CH₂F, CH₂CF₃.

Unter dem Begriff "C₆₋₁₀-Aryl" (auch soweit sie Bestandteil anderer Reste sind) werden aromatische Ringsysteme mit 6 oder 10 Kohlenstoffatomen verstanden. Beispielsweise werden hierfür genannt: Phenyl oder Naphthyl, bevorzugter Arylrest ist Phenyl. Soweit nicht anders beschreiben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "heterocyclische Ringe" oder "Heterocyclus" werden vier-, fünf-, sechs-oder siebengliedrige, gesättigte,ungesättigte oder teilweise ungesättigte monocyclische heterocyclische Ringe oder sieben-, acht-, neun- oder zehngliedrige, heterocyclische Ringe verstanden die ein, zwei oder drei Heteroatome, ausgewählt aus der Gruppe Sauerstoff, Schwefel und Stickstoff enthalten können. Dabei kann der Ring über ein Kohlenstoffatom oder, falls vorhanden, über ein Stickstoffatom mit dem Molekül verknüpft sein. Als Beispiele für fünf-, sechs- oder siebengliedrige, gesättigte, ungesättigte oder teilweise ungesättigte Heterocyclen werden genannt:

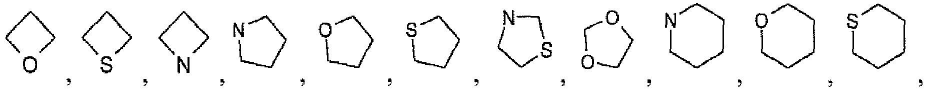

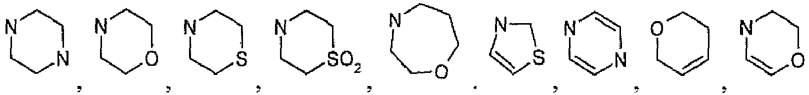

Soweit nicht anders erwähnt, kann ein heterocyclischer Ring mit einer Ketogruppe versehen sein. Als Beispiel hierfür werden genannt.

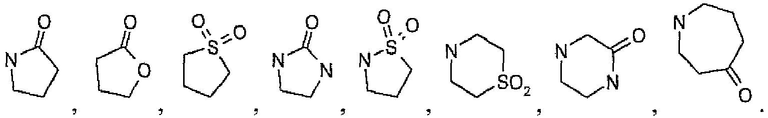

Als Beispiel für sieben-, acht-, neun-, oder zehngliedrige gesättigte, ungesättigte oder teilweise ungesättigte bicyclische Heterocyclen werden genannt Pyrrolizin, Indol, Indolizin, Isoindol, Indazol, Purin, Chinolin, Isochinolin, Benzimidazol, Benzofuran, Benzopyran, Benzothiazol, Benzoisothiazol, Pyridopyrimidin, Pteridin, Pyrimidopyrimidin,

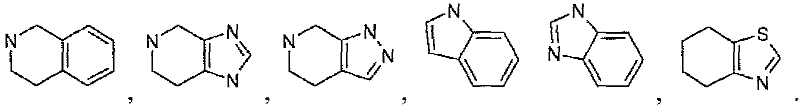

Obwohl unter dem Begriff "heterocyclische Ringe" oder "Heterocyclus" umfasst, definiert der Begriff "Heteroaromat" oder "Heteroaryl" fünf- oder sechsgliedrige heterocyclische monocyclische Aromaten oder 5-10 gliedrige, bicyclische Heteroarylringe, die ein, zwei oder drei Heteroatome ausgewählt aus der Gruppe Sauerstoff, Schwefel und Stickstoff, enthalten können und so viele konjugierte Doppelbindungen enthalten, dass ein aromatisches System gebildet wird. Der Ring kann über ein Kohlenstoffatom oder -falls vorhanden- über ein Stickstoffatom mit dem Molekül verknüpft sein. Als Beispiele für fünf- oder seclisgliedrige Heteroaryle werden genannt:

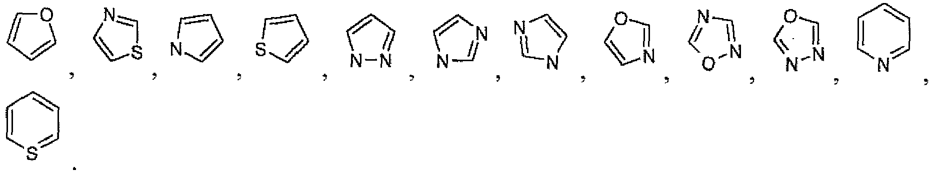

### BEISPIELE

Die erfindungsgemäßen Verbindungen können nach an sich aus der Literatur bekannten Methoden hergestellt werden, wie sie zum Beispiel in DE 3540952 beschrieben sind.

Die erfindungsgemäßen Verbindungen werden nach den Schemata 1 bis 3 hergestellt. Nach 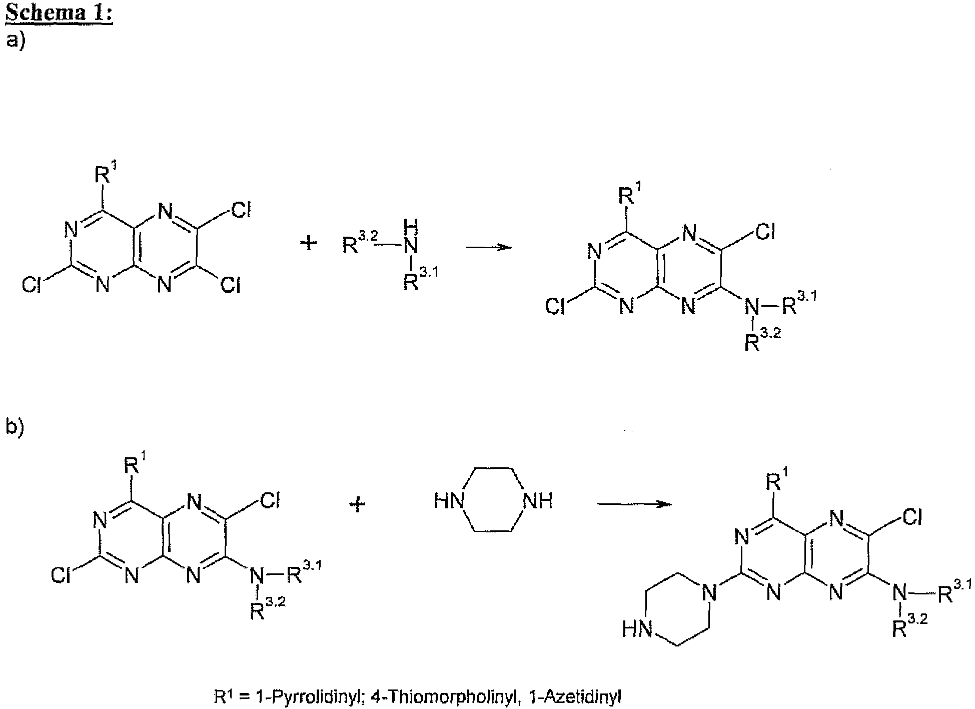 wurden die folgenden Beispiel-Verbindungen hergestellt:

### Beispiel 1: 7-Ammo-6-chlor-2-piperazin-1-yl-4-pyrrolidin-1-yl-pteridin (Beispiel-Verbindung 99 aus Tabelle 1, nach Schema 1 hergestellt)

a) 7-Amino-2,6-dichlor-4-pyrrolidin-1-yl-pteridin: 500 mg (1,6 mmol) 4-Pyrrolidin-1-yl-2,6,7-trichlor-pteridin werden mit 10 ml Dioxan und 15 ml einer 0,5 molaren Lösung von Ammoniak in Dioxan versetzt und ca. 16 h bei 60° C gerührt. Der Ansatz wird mit Wasser versetzt und mit Dichlormethan extrahiert. Die organische Phase wird mit Wasser gewaschen, über Natriumsulfat getrocknet und im Vakuum vom Lösungsmittel befreit. Der Rückstand wird ohne weitere Reinigung im nächsten Schritt eingesetzt.
b) 7-Amino-6-chlor-2-piperazin-1-yl-4-pyrrolidin-1-yl-pteridin: Der Rückstand aus a) wird in 15 ml Dioxan gelöst und langsam zu einer auf 80° C erhitzten Lösung von 0,707 g (8 mmol) Piperazin in 10 ml Dioxan gegeben. Man rührt eine Stunde weiter, dann wird der Ansatz mit Wasser versetzt und mit Dichlormethan extrahiert. Die organische Phase wird mit Wasser gewaschen, über Natriumsulfat getrocknet und im Vakuum vom Lösungsmittel befreit. Der Rückstand wird chromatographisch gereinigt, Ausbeute 410 mg (56% d. Th.).

### Beispiel 2: 6-Chlor-7-cyclobutylamino-2-piperazin-1-yl-4-pyrrolidin-1-yl-pteridin (Beispiel-Verbindung 76 aus Tabelle 1, nach Schema 1 hergestellt):

a) 7-Cyclobutylamino-2,6-dichlor-4-pyrrolidin-1-yl-pteridin: 250 mg (0,82 mmol) 4-Pyrrolidin-1-yl-2,6,7-trichlor-pteridin werden in 25 ml Tetrahydrofuran suspendiert und mit 70 µl (0,82 mmol) Cyclobutylamin und 175 µl (1 mmol) Diisoproyplethylamin versetzt. Man rührt ca. 16 h bei Raumtemperatur, versetzt mit 20 ml Wasser und extrahiert mit 2 mal 20 ml Dichlormethan. Die organische Phase wird über Natriumsulfat getrocknet und im Vakuum vom Lösungsmittel befreit. Der Rückstand wird ohne weitere Reinigung im nächsten Schritt eingesetzt.
b) 6-Chlor-7-cyclobutylamino-2-piperazin-1-yl-4-pyrrolidin-1-yl-pteridin: 360 mg (4,2 mmol) Piperazin werden in 15 ml Dioxan suspendiert und auf 80° C erhitzt. Zu dieser Lösung tropft man langsam eine Lösung von 285 mg (0,84 mmol) 7-Cyclobutylamino-2,6-dichlor-4-pyrrolidin-1-yl-pteridin in 15 ml Dioxan. Man rührt noch ca. 16 h bei 80° C weiter und befreit dann das Reaktionsgemisch im Vakuum vom Lösungsmittel. Der Rückstand wird chromatographisch gereinigt, der erhaltene Feststoff mit Diisopropylether verrieben, abgenutscht und getrocknet. Ausbeute 192 mg (59% d. Th.).

### Beispiel 3: 6-Chlor-7-((R)-2-hydroxypropyl)amino-)-2-piperazin-1-yl-4-pyrrolidin-1-yl-pteridin (Beispiel-Verbindung 24 aus Tabelle 1- nach Schema 1 hergestellt):

a) 2.6-Dichlor-7-((R)-2-hydroxypropyl)amino-4-pyrrolidin-1-yl-pteridin: 300 mg (0,99 mmol) 4-Pyrrolidin-1-yl-2,6,7-trichlor-pteridin werden in 15 ml Dioxan suspendiert und mit 121 mg (1,6 mmol) (R)-1-Amino-propan-2-ol und 185 µl (1,4 mmol) Diisopropylethylamin versetzt. Man rührt ca. 16 h bei 40° C, versetzt mit 20 ml Wasser und extrahiert mit 2 mal 20 ml Dichlormethan. Die organische Phase wird über Natriumsulfat getrocknet und im Vakuum vom Lösungsmittel befreit. Der Rückstand wird ohne weitere Reinigung im nächsten Schritt eingesetzt.
b) 6-Chlor-7-((R)-2-hydroxypropyl)amino-2-piperazin-1-yl-4-pyrrolidin-1-yl-pteridin: 440 mg (5,1 mmol) Piperazin werden in 15 ml Dioxan suspendiert und auf 80° C erhitzt. Zu dieser Lösung tropft man langsam eine Lösung von 349 mg (1 mmol) 2,6-Dichlor-7-((R)-2-hydroxypropyl)amino-4-pyrrolidin-1-yl-pteridin in 15 ml Dioxan. Man rührt noch ca. 16 h bei 80° C weiter und befreit dann das Reaktionsgemisch im Vakuum vom Lösungsmittel. Der Rückstand wird chromatographisch gereinigt, mit Essigester und Petrolether kristallisiert, abgenutscht und getrocknet. Ausbeute 194 mg (49% d. Th.).

### Beispiel 4: 7-(1,1-Bis-(hydroxymethyl)propyl)amino-6-chlor-2piperazin-1-yl-4-pyrrolidin-1-yl-pteridin (Beispiel-Verbindung 33 aus Tabelle 1, nach Schema 1 hergestellt) :

a) 7-(1,1-Bis-(hydroxymethyl)propyl)amino-2,6-dichlor-4-pyrrolidin-1-yl-pteridin: 65 mg (1 mmol) 1,1-Bis-(hydroxymethyl)propyl)-amin werden in 4 ml Tetrahydrofuran gelöst und bei -10° C mit 0,27 ml (0,55 mmol) einer 2 molaren Lösung von Lithiumdiisopropylamid in Tetrahydrofuran versetzt. Man rührt eine Stunde bei Raumtemperatur und kühlt wieder auf -10° C ab. Anschließend wird eine Suspension aus 167 mg (0,55 mmol) 4-Pyrrolidin-1-yl-2,6,7-trichlor-pteridin in 15 ml Dioxan zugetropft. Man lässt auf Raumtemperatur kommen und rührt ca. 16 h weiter. Das Reaktionsgemisch wird mit Wasser versetzt und mit Dichlormethan extrahiert. Die organische Phase wird über Natriumsulfat getrocknet und im Vakuum vom Lösungsmittel befreit. Der Rückstand wird chromatographisch gereinigt, mit Ether verrieben und abgesaugt. Ausbeute 66 mg (31% d. Th.)
b) 7-(1,1-Bis-(hydroxymethyl)propyl)amino-6-chlor-2-piperazin-1-yl-4-pyrrolidin-1-yl-pteridin: 1,02 g (12 mmol) Piperazin werden in 45 ml Dioxan suspendiert und auf 80° C erhitzt. Zu dieser Lösung tropft man langsam eine Lösung von 920 mg (2,4 mmol) 7-(1,1-Bis-(hydroxymethyl)propyl)amino-2,6-dichlor-4-pyrrolidin-1-yl-pteridin in 100 ml Dioxan. Man rührt ca. 16 h bei 80° C und gießt dann das Reaktionsgemisch auf 30 ml Eiswasser. Man extrahiert mit Dichlormethan, die organische Phase wird über Natriumsulfat getrocknet und im Vakuum vom Lösungsmittel befreit. Der Rückstand wird in etwas Dichlormethan aufgenommen und mit Ether versetzt, der entstehende Niederschlag wird abgesaugt. Ausbeute 1.032 g (99% d. Th.).

### Beispiel 5:6-Chlor-7-(4,5-dimethylthiazol-2-yl)-amino-2-piperazin-1-yl-4-pyrrolidin-1-yl-pteridin (Beispiel-Verbindung 91 aus Tabelle 1, nach Schema 1 hergestellt):

a+b) 100 mg (0,33 mmol) 4-Pyrrolidin-1-yl-2,6,7-trichlor-pteridin werden in 5 ml Dioxan gelöst und mit 46 mg (0,36 mmol) 2-Amino-4,5-dimethyl-thiazol sowie 50 mg (0,36 mmol) Kaliumcarbonat versetzt. Man rührt 3 Stunden bei 40° C, saugt den entstandenen Niederschlag ab und nimmt ihn in Dioxan auf. Diese Lösung tropft man langsam zu einer auf 80° C erhitzten Lösung von 141 mg (2 mmol) Piperazin in 15 ml Dioxan. Man rührt ca. 16 h bei 80° C, lässt abkühlen und gießt das Reaktionsgemisch auf ca. 30 ml Eiswasser. Man extrahiert mit Dichlormethan, trocknet die organische Phase über Natriumsulfat und entfernt das Lösungsmittel im Vakuum. Der Rückstand wird chromatographisch gereinigt, Ausbeute 17 mg (11% d. Th.).

### Beispiel 6: 6-Chlor-7-(5-methylthiazol-2-yl)-amino-2-piperazin-1-yl-4-pyrrolidin-1-yl-pteridin (Beispiel-Verbindung 92 aus Tabelle 1. nach Schema 1 hergestellt):

a+b) 100 mg (0,33 mmol) 4-Pyrrolidin-1-yl-2,6,7-trichlor-pteridin werden in 5 ml Dioxan gelöst und mit 41 mg (0,36 mmol) 2-Amino-5-methylthiazol sowie 41 mg (0,36 mmol) Kalium-tert.-butanolat versetzt. Man rührt 3 h bei 40° C, lässt abkühlen und tropft dann das Reaktionsgemisch langsam zu einer auf 80° C erhitzten Lösung von 141 mg (2 mmol) Piperazin in 15 ml Dioxan. Man rührt ca. 16 h bei 80° C, kühlt ab und entfernt das Lösungsmittel im Vakuum. Der Rückstand wird chromatographisch gereinigt, Ausbeute 5,5 mg (3% d. Th.).

### Beispiel 7: 6-Chlor-7-(4-methylthiazol-2-yl)-amino-2-piperazin-1-yl-4-pyrrolidin-1-yl-pteridin (Beispiel-Verbindung 96 nach Tabelle 1, nach Schema 1 hergestellt):

a) 2,6-Dichlor-7-(4-methylthiazol-2-yl)-amino-4-pyrrolidin-1-yl-pteridin: 94 mg (0,82 mmol) 2-Amino-4-methylthiazol werden in 15 ml Tetrahydrofuran gelöst und bei -10° C mit 410 µl (0,82 mmol) einer 2 molaren Lösung von Lithiumdiisopropylamid in Tetrahydrofuran versetzt. Man rührt eine Stunde bei Raumtemperatur, kühlt wieder auf -10° C ab und tropft eine Suspension von 250 mg (0,82 mmol) 4-Pyrrolidin-1-yl-2,6,7-trichlor-pteridin in 10 ml Tetrahydrofuran zu. Man lässt auf Raumtemperatur kommen und rührt ca. 16h weiter. Man versetzt mit Wasser und extrahiert mit Essigester. Die organische Phase wird über Natriumsulfat getrocknet und im Vakuum vom Lösungsmittel befreit. Der Rückstand wird mit Acetonitril verrieben, der entstandene Niederschlag wird abgesaugt und ohne weitere Reinigung im nächsten Schritt eingesetzt, Ausbeute 105 mg (33% d. Th.).
b) 6-Chlor-7-(4-methylthiazol-2-yl)-amino-2-piperazin-1-yl-4-pyrrolidin-1-yl-pteridin: 118 mg (1,37 mmol) Piperazin werden in 10 ml Dioxan suspendiert und auf 80° C erhitzt. Man tropft eine Lösung von 105 mg (0,28 mmol) 2,6-Dichlor-7-(4-methylthiazol-2-yl)-amino-4-pyrrolidin-1-yl-pteridin in 5 ml Dioxan zu und rührt für ca. 16 h bei 80° C weiter. Das Reaktionsgemisch wird auf Eiswasser gegeben und mit Dichlormethan extrahiert. Die organische Phase wird über Natriumsulfat getrocknet und im Vakuum vom Lösungsmittel befreit, der Rückstand wird mit Ether verrieben und abgesaugt. Ausbeute 54 mg (46% d. Th.).

### Beispiel 8: 6-Chlor-7-(3-cyclopropyl-1-hydroxy-prop-2-yl)-amino-2-piperazin-2-yl-4-pyrrolidin-1-yl-pteridin (Beispiel-Verbindung 110 aus Tabelle 1, nach Schema 1 hergestellt):

a) + b) 200 mg (0,66 mmol) 4-Pyrrolidin-1-yl-2,6,7-trichlor-pteridin werden in 10 ml Dioxan gelöst und mit 83,4 mg (0,72 mmol) 2-Amino-3-cyclopropyl-propan-1-ol und 121 µl (0,92 mmol) Diisopropylethylamin versetzt. Man rührt ca. 16 h bei 40° C. Das Reaktionsgemisch wird auf Raumtemperatur abgekühlt, mit Wasser versetzt und mit Dichlormethan extrahiert. Die organische Phase wird getrocknet und im Vakuum vom Lösungsmittel befreit. Der Rückstand wird chromatographisch gereinigt. Man erhält 90 mg 7-(3-cyclopropyl-1-hydroxy-prop-2-yl)-amino-2,6-dichlor-4-pyrrolidin-1-yl-pteridin. 70 mg dieser Substanz werden in 11 ml Dioxan gelöst werden. Diese Lösung wird langsam zu einer 80° C heißen Lösung von 78,9 mg (0,92 mmol) Piperazin in 11 ml Dioxan getropft. Es wird ca. 16 h bei 80° C gerührt, auf Raumtemperatur abgekühlt und dann auf Eiswasser gegossen. Man extrahiert mit Dichlormethan, die organische Phase wird über Natriumsulfat getrocknet und im Vakuum vom Lösungsmittel befreit. Der Rückstand wird chromatographisch gereinigt. Ausbeute 79 mg (100% d. Th.).

### Beispiel 9: 4-Azetidin-1-yl-6-chlor-7-cyclobutylamino-2-piperazin-1-yl-pteridin (Beispiel-Verbindung 113 aus Tabelle 1, nach Schema 1 hergestellt):

a) + b) 100 mg (0,34 mmol) 4-Azetidin-1-yl-2,6,7-trichlor-pteridin werden in 5 ml Dioxan gelöst und mit 30 µl (0,35 mmol) Cyclobutylamin und 66 µl (0,39 mmol) Diisopropylethylamin versetzt. Man rührt ca. 16 h bei 40° C. Das Reaktionsgemisch wird auf Raumtemperatur abgekühlt und im Vakuum vom Lösungsmittel befreit. Der Rückstand wird chromatographisch gereinigt (reversed phase). Man erhält 60 mg (54% der Theorie) 4-Azetidin-1-yl-7-cyclobutylamino-2,6-dichlor-pteridin. Diese Substanz wird in 5 ml Dioxan gelöst und langsam zu einer 80° C heißen Lösung von 80 mg (0,93 mmol) Piperazin in 5 ml Dioxan getropft. Es wird ca. eine Stunde bei 80° C gerührt, auf Raumtemperatur abgekühlt und dann mit Wasser versetzt. Man extrahiert mit Dichlormethan, die organische Phase wird über Natriumsulfat getrocknet und im Vakuum vom Lösungsmittel befreit. Ausbeute 50 mg (72% d. Th.).

### Beispiel 10: 6-Chlor-7-(1-Hydroxy-cyclohexylmethyl)-amino-2-piperazin-1-yl-4-pyrrolidin-1-yl-pteridin (Beispiel-Verbindung 116 aus Tabelle 1, nach Schema 1 hergestellt):

a) + b) 100 mg (0,33 mmol) 4-Pyrrolidin-1-yl-2,6,7-trichlor-pteridin werden in 5 ml Dioxan gelöst und mit 60 mg (0,36 mmol) 1-Aminomethyl-1-cyclohexanol und 108 µl (0,82 mmol) Diisopropylethylamin versetzt. Man rührt ca, 70 h bei 40° C. Das Reaktionsgemisch wird dann zu einer 80° C heißen Lösung von 141 mg (1,64 mmol) Piperazin in 15 ml Dioxan getropft. Es wird ca. 16 Stunden bei 80° C gerührt, auf Raumtemperatur abgekühlt und dann mit Wasser versetzt. Man extrahiert mit Dichlormethan, die organische Phase wird über Natriumsulfat getrocknet und im Vakuum vom Lösungsmittel befreit. Der Rückstand wird mit Diethylether verrieben, der entstandene Feststoff abgesaugt, mit Ether gewaschen und getrocknet. Ausbeute 100 mg (68% d. Th.).

### Beispiel 11: 6-Chlor-7-(N-methyl-2-hydroxyethyl)-amino-2-piperazin-1-yl-4-pyrrolidin-1-yl-pteridin (Beispiel-Verbindung 117 aus Tabelle 1, nach Schema 1 hergestellt):

a) + b) 100 mg (0,33 mmol) 4-Pyrrolidin-1-yl-2,6,7-trichlor-pteridin werden in 5 ml Dioxan gelöst und mit 27,1 mg (0,36 mmol) 2-Methylamino-ethanol und 61 µl (0,36 mmol) Diisopropylethylamin versetzt. Man rührt ca. 70 h bei 40° C. Das Reaktionsgemisch wird auf Raumtemperatur abgekühlt, mit Wasser versetzt und mit Dichlormethan extrahiert. Die organische Phase wird getrocknet und im Vakuum vom Lösungsmittel befreit. Der Rückstand wird chromatographisch gereinigt. Man erhält 100 mg 2,6-Dichlor-7-(N-methyl-2-hydroxyethyl)-amino-4-pyrrolidin-1-yl-pteridin welches in 5 ml Dioxan gelöst wird und langsam zu einer 80° C heißen Lösung von 141 mg (1,64 mmol) Piperazin in 15 ml Dioxan getropft wird. Es wird ca. 16 Stunden bei 80° C gerührt, auf Raumtemperatur abgekühlt und dann mit Wasser versetzt. Man extrahiert mit Dichlormethan, die organische Phase wird über Natriumsulfat getrocknet und im Vakuum vom Lösungsmittel befreit. Der Rückstand wird chromatographisch gereinigt. Ausbeute 130 mg (enthält noch Lösungsmittel).

Alle Beispiel-Verbindungen aus Tabelle 1, die mit "Schema 1" gekennzeichnet sind, werden analog des Schemas 1 und analog der obigen ausführlichen Synthesevorschriften hergestellt.

Nach 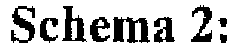 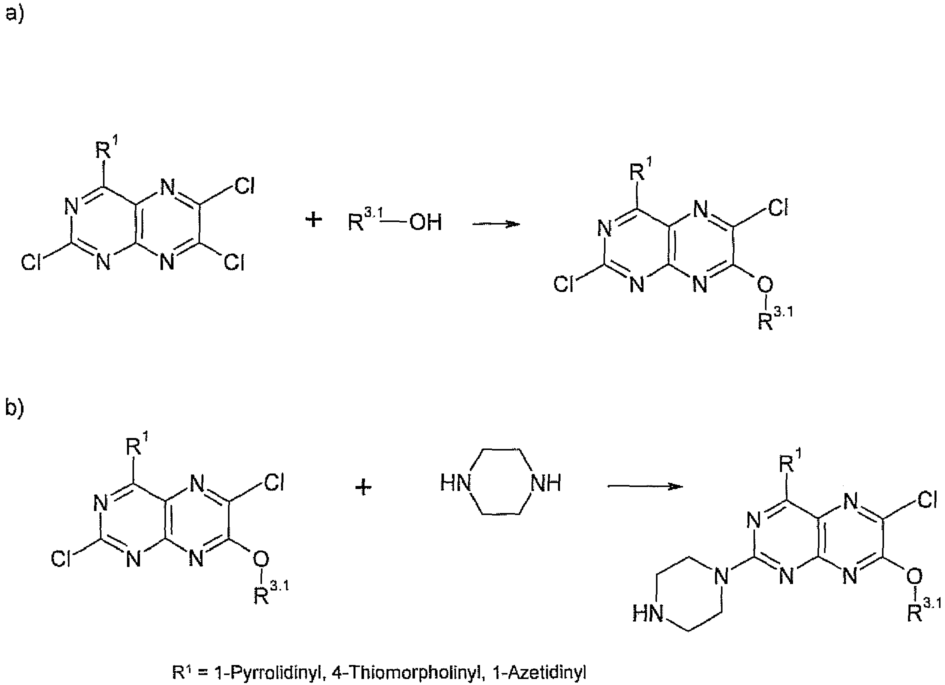 wurden die nachfolgenden Beispiel-Verbindungen hergestellt:

### Beispiel 12: 6-Chlor-7-cyclopropylmethyloxy-2-piperazin-1-yl-4-pyrrolidin-1-yl-pteridin (Beispiel-Verbindung 81 aus Tabelle 1, nach Schema 2 hergestellt):

a) 7-Cyclopropylmethylmethyloxy-2,6-dichlor-4-pyrrolidin-1-yl-pteridin: 0,067 ml (0,82 mmol) Cyclopropylmethanol werden in 12 ml Tetrahydrofuran gelöst und unter Argon mit 0,41 ml (0,82 mmol) einer 2 molaren Lösung von Lithiumdiisopropylamid in Tetrahydrofuran versetzt. Man rührt 30 min bei Raumtemperatur, anschließend wird die Mischung langsam zu einer auf -10° C gekühlten Lösung von 250 mg (0,82 mmol) 4-Pyrrolidin-1-yl-2,6,7-trichlor-pteridin in 12 ml Tetrahydrofuran gegeben. Man rührt eine Stunde bei -10° C, lässt langsam auf Raumtemperatur kommen und versetzt mit 25 ml Wasser. Das Reaktionsgemisch wird mit Dichlormethan extrahiert, die organische Phase wird über Natriumsulfat getrocknet und im Vakuum vom Lösungsmittel befreit. Der Rückstand wird chromatographisch gereinigt, Ausbeute 141 mg (51 % d. Th.)
b) 6-Chlor-7-cyclopropylmethyloxy-2-piperazin-1-yl-4-pyrrolidin-1-yl-pteridin: Zu einer Lösung von 139 mg (0,41 mmol) 7-Cyclopropylmethyloxy-2,6-dichlor-4-pyrrolidin-1-yl-pteridin in 15 ml Tetrahydrofuran wird langsam bei Raumtemperatur eine Lösung von 158 mg (1,8 mmol) Piperazin in 10 ml Tetrahydrofuran getropft. Anschließend wird für ca. 16 h am Rückfluss gekocht. Das Reaktionsgemisch wird im Vakuum vom Lösungsmittel befreit, der Rückstand wird chromatographisch gereinigt, Ausbeute 120 mg (75% d. Th.)

### Beispiel 13: 6-Chlor-7-((R)-3-tetrahydrofuryl)-oxy-2-piperazin-1-yl-4-thiomorpholin-4-yl-pteridin (Beispiel-Verbindung 56 aus Tabelle 1, nach Schema 2 hergestellt):

a) + b) 65 mg (0,74 mmol) (R)-3-Hydroxyptetrahydrofuran werden in 3 ml Tetrahydrofuran gelöst und unter Schutzgas bei -10° C mit 371 µl (0,74 mmol) einer 2 molaren Lösung von Lithiumdiisopropylamin in Tetrahydrofuran versetzt. Man lässt langsam auf Raumtemperatur kommen und rührt eine Stunde weiter. Man kühlt wieder auf -10° C und gibt langsam eine Suspension von 250 mg (0,74 mmol) 4-Thiomorpholin-4-yl-2,6,7-trichlor-pteridin in 9 ml Tetrahydrofuran zu. Man rührt 1 Stunde bei -10° C weiter und lässt dann langsam auf Raumtemperatur kommen. Das Reaktionsgemisch wird mit Wasser versetzt und mit Dichlormethan extrahiert. Die organische Phase wird über Natriumsulfat getrocknet und im Vakuum vom Lösungsmittel befreit. Der Rückstand wird in 10 ml Dioxan aufgenommen und langsam zu 80° C heißen Lösung von 320 mg (3,7 mmol) Piperazin in 5 ml Dioxan getropft. Man rührt eine Stunde bei 80° C weiter, kühlt auf Raumtemperatur ab und versetzt das Reaktiongemisch mit Wasser. Man extrahiert mit Dichlormethan, die organische Phase wird über Natriumsulfat getrocknet und im Vakuum vom Lösungsmittel befreit. Der Rückstand wird chromatographisch gereinigt. Ausbeute 142 mg (35% d. Th.)

### Beispiel 14: 6-Chlor-7-(2.2-difluorethyloxy)-2-piperazin-1-yl-4-pyrrolidin-1-yl-pteridin (Beispiel-Verbindung 104 aus Tabelle 1, nach Schema 2 hergestellt):

a) + b) 50 µl (0,82 mmol) 2,2-Difluorethanol werden in 12 ml Tetrahydrofuran gelöst und unter Schutzgas mit 410 µl (0,82 mmol) einer 2 molaren Lösung von Lithiumdiisopropylamin in Tetrahydrofuran versetzt. Man rührt 30 Minuten und gibt das Gemisch dann zu einer auf -10° C gekühlten Lösung von 250 mg (0,82 mmol) 4-Pyrrolidin-1-yl-2,6,7-trichlor-pteridin in 12 ml Tetrahydrofuran. Man rührt eine Stunde bei -10° C, lässt langsam auf Raumtemperatur kommen und rührt vier Stunden weiter. Das Lösungsmittel wird im Vakuum entfernt, der Rückstand mit 50 ml Wasser versetzt und mit Dichlormethan extrahiert. Die organische Phase wird über Natriumsulfat getrocknet und im Vakuum vom Lösungsmittel befreit. Der Rückstand wird in 15 ml Tetrahydrofuran aufgenommen und mit einer Lösung von 313 mg (3,6 mmol) Piperazin in 10 ml Tetrahydrofuran versetzt. Man kocht ca. 16 Stunden unter Rückfluss, kühlt auf Raumtemperatur ab und entfernt das Lösungsmittel im Vakuum. Der Rückstand wird mit Wasser versetzt und mit Dichlormethan extrahiert. Die organische Phase wird über Natriumsulfat getrocknet und im Vakuum vom Lösungsmittel befreit. Man versetzt mit Diisopropylether und erhält einen gelblichen Feststoff. Ausbeute 225 mg (70% d. Th.)

### Beispiel 15 6-Chlor-7-hydroxy-2-piperazin-1-yl-4-pyrrolidin-1-yl-pteridin (Beispiel-Verbindung 105 aus Tabelle 1, nach Schema 2 hergestellt):

a) + b) Zu einer auf -10° C gekühlten Lösung von 250 mg (0,82 mmol) 4-Pyrrolidin-1-yl-2,6,7-trichlor-pteridin in 15 ml Tetrahydrofuran tropft man 460 µl (0,82 mmol) einer 10%igen Lösung von Kaliumhydroxid in Wasser. Man rührt eine Stunde bei -10° C, lässt langsam auf Raumtemperatur kommen und rührt ca. 16 Stunden weiter. Dann wird eine Lösung von 318 mg (3,69 mmol) Piperazin in 10 ml Tetrahydrofuran zugegeben und die Mischung für ca. 16 Stunden unter Rückfluss gekocht. Anschließend wird das Lösungsmittel im Vakuum entfernt, der Rückstand mit Wasser versetzt und mit Dichlormethan extrahiert. Die organische Phase wird über Natriumsulfat getrocknet und im Vakuum vom Lösungsmittel befreit. Der Rückstand wird chromatographisch gereinigt. Ausbeute 50 mg (14% d. Th.)

### Beispiel 16: (R)-6-Chlor-7-(3-tetrahydropyranyloxy)-2-piperazin-1-yl-4'-pyrrolidin-1-yl-pteridin (Beispiel-Verbindung 112 aus Tabelle 1, nach Schema 2 hergestellt):

a) + b) 170 mg (1,67 mmol) (R)-3-Hydroxytetrahydropyran werden in 5 ml Tetrahydrofuran gelöst und unter Schutzgas bei -10° C mit 821 µl (1,64 mmol) einer 2 molaren Lösung von Lithiumdiisopropylamin in Tetrahydrofuran versetzt. Man rührt 30 Minuten bei -10° C, dann eine Stunde bei Raumtemperatur. Die Mischung wird wieder auf -10° C abgekühlt und mit einer Suspension aus 500 mg (1,64 mmol) 4-Pyrrolidin-1-yl-2,6,7-trichlor-pteridin in 15 ml Tetrahydrofuran versetzt. Man rührt eine Stunde bei -10° C, lässt langsam auf Raumtemperatur kommen und rührt vier Stunden weiter. Die Reaktionsmischung wird mit ca. 100 ml Wasser versetzt und mit Dichlormethan extrahiert. Die organische Phase wird über Natriumsulfat getrocknet und im Vakuum vom Lösungsmittel befreit. Der Rückstand wird in ca. 50 ml Diethylether aufgenommen und eine Stunde durchgeführt. Der Feststoff wird abgesaugt, mit Diethylether gewaschen und getrocknet. Ausbeute 320 mg (53% der Theorie). Das so erhaltene (R)-2,6-Dichlor-7-(3-tetrahydropyranyloxy)-4-pyrrolidin-1-yl-pteridin wird in 13 ml Dioxan gelöst und langsam zu einer 80° C heißen Lösung von 372 mg (4,3 mmol) Piperazin in 12 ml Dioxan getropft. Man rührt ca. 16 Stunden bei 80° C weiter, kühlt auf Raumtemperatur ab und versetzt das Reaktiongemisch mit Wasser. Man extrahiert mit Dichlormethan, die organische Phase wird über Natriumsulfat getrocknet und im Vakuum vom Lösungsmittel befreit. Der Rückstand wird chromatographisch gereinigt. Das erhaltene Produkt wird mit Diethylether verrührt, die Festsubstanz abgesaugt, mit Diethylether gewaschen und getrocknet. Ausbeute 170 mg (47% d. Th.)

### Beispiel 17: 4-Azetidin-1-yl-6-Chlor-7-(tetrahydropyran-4-yloxy)-2-piperazin-1-yl-pteridin (Beispiel-Verbindung 114 aus Tabelle 1, nach Schema 2 hergestellt):

a) + b) 56 µl (0,59 mmol) 4-Hydroxytetrahydropyran werden in 2 ml Tetrahydrofuran gelöst und unter Schutzgas bei -10° C mit 293 µl (0,59 mmol) einer 2 molaren Lösung von Lithiumdiisopropylamin in Tetrahydrofuran versetzt. Man rührt 30 Minuten bei -10° C, dann eine Stunde bei Raumtemperatur. Die Mischung wird wieder auf -10° C abgekühlt und mit einer Suspension aus 170 mg (0,59 mmol) 4-Azetidin-1-yl-2,6,7-trichlor-pteridin in 5 ml Tetrahydrofuran versetzt. Man rührt eine Stunde bei -10° C, lässt langsam auf Raumtemperatur kommen und rührt ca. 16 Stunden weiter. Die Reaktionsmischung wird mit Wasser versetzt und mit Dichlormethan extrahiert. Die organische Phase wird über Natriumsulfat getrocknet und im Vakuum vom Lösungsmittel befreit. Der Rückstand wird chromatographisch (reversed phase) gereinigt. Man erhält 90 mg 4-Azetidin-1-yl-2,6-dichlor-7-(tetrahydropyran-4-yloxy)-pteridin, das in 5 ml Dioxan gelöst und langsam zu einer 80° C heißen Lösung von 109 mg (1,27 mmol) Piperazin in 5 ml Dioxan getropft wird. Man rührt ca. 1 Stunde bei 80° C weiter, kühlt auf Raumtemperatur ab und versetzt das Reaktiongemisch mit Wasser. Man extrahiert mit Dichlormethan, die organische Phase wird über Natriumsulfat getrocknet und im Vakuum vom Lösungsmittel befreit. Ausbeute 70 mg (68% d. Th.)

### Beispiel 18: (R)-4-Azetidin-1-yl-6-chlor-7-(tetrahydrofuran-3-yl-oxy)-2-piperazin-1-yl-pteridin (Beispiel-Verbindung 115 aus Tabelle 1, nach Schema 2 hergestellt):

a) + b) 111 µl (1,38 mmol) (R)-3-Hydroxytetrahydrofuran werden in 5 ml Tetrahydrofuran gelöst und unter Schutzgas bei -10° C mit 688 µl (1,38 mmol) einer 2 molaren Lösung von Lithiumdiisopropylamin in Tetrahydrofuran versetzt. Man rührt 30 Minuten bei -10° C, dann eine Stunde bei Raumtemperatur. Die Mischung wird wieder auf-10° C abgekühlt und mit einer Suspension aus 400 mg (1,38 mmol) 4-Azetidin-1-yl-2,6,7-trichlor-pteridin in 10 ml Tetrahydrofuran versetzt. Man rührt eine Stunde bei -10° C, lässt langsam auf Raumtemperatur kommen und rührt ca. 16 Stunden weiter. Die Reaktionsmischung wird mit Wasser versetzt und mit Dichlormethan extrahiert. Die organische Phase wird über Natriumsulfat getrocknet und im Vakuum vom Lösungsmittel befreit. Der Rückstand wird mit Diethylether verrührt, der ausgefallene Feststoff wird abgesaugt, mit Diethylether gewaschen und getrocknet. Man erhält 290 mg (R)-4-Azetidin-1-yl-2,5-dichlor-7-(tetrahydrofuran-3-yl-oxy)-pteridin, das in 13 ml Dioxan gelöst und langsam zu einer 80° C heißen Lösung von 365 mg (4,24 mmol) Piperazin in 12 ml Dioxan getropft wird. Man rührt ca. 16 Stunden bei 80° C weiter, kühlt auf Raumtemperatur ab und versetzt das Reaktiongemisch mit Wasser. Man extrahiert mit Dichlormethan, die organische Phase wird über Natriumsulfat getrocknet und chromatographisch gereinigt. Ausbeute 170 mg (51% d. Th.)

Alle Beispiel-Verbindungen aus Tabelle 1, die mit "Schema 2" gekennzeichnet sind, werden analog des Schemas 2 und analog der obigen ausführlichen Synthesevorschriften hergestellt.

Nach 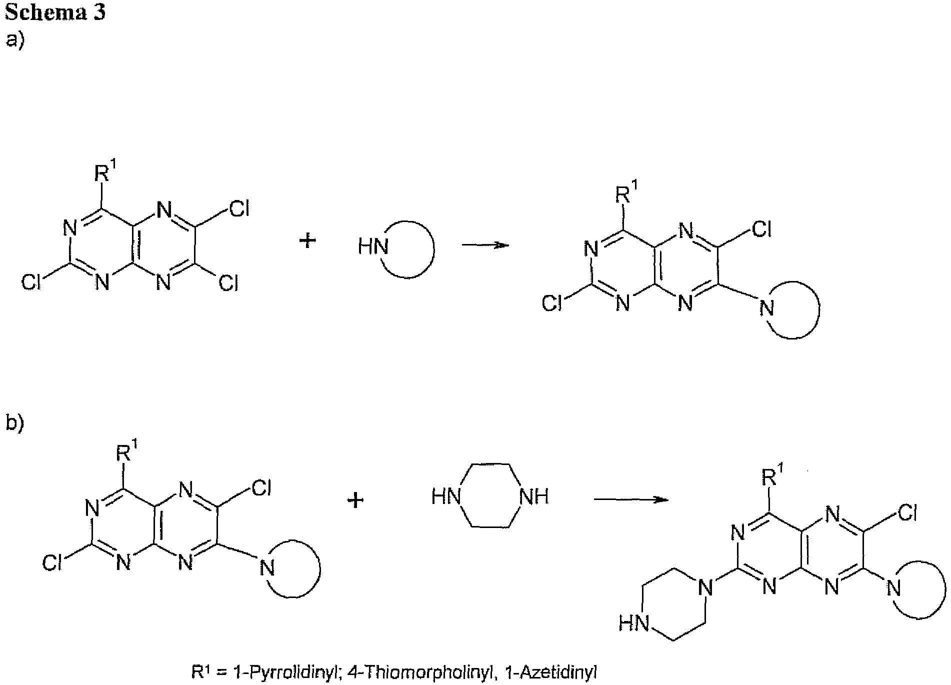 wurden die nachfolgenden Beispiel-Verbindungen hergestellt:

### Beispiel 19: 6-Chlor-7-(4-hydroxypiperidin-1-yl)-2-piperazin-1-yl-4-pyrrolidin-1-yl-pteridin (Beispiel-Verbindung 64 aus Tabelle 1, nach Schema 3 hergestellt):

a) + b) 80 mg (0,26 mmol) 4-Pyrrolidin-1-yl-2,6,7-trichlor-pteridin werden in 5 ml Dioxan gelöst und mit 29 mg (0,29 mmol) 4-Hydroxypiperidin und 49 µl (0,37 mmol) Diisopropylethylamin versetzt. Man rührt ca. 16 h bei 40° C. Das Reaktionsgemisch wird anschließend langsam zu einer 80° C heißen Lösung von 113 mg (1 mmol) Piperazin in 15 ml Dioxan getropft. Es wird ca. 16 h bei 80° C gerührt, auf Raumtemperatur abgekühlt und dann auf Eiswasser gegossen. Man extrahiert mit Dichlormethan, die organische Phase wird über Natriumsulfat getrocknet und im Vakuum vom Lösungsmittel befreit. Der Rückstand wird mit etwas Dichlormethan aufgenommen und mit Petrolether versetzt, der entstandene Niederschlag wird abgesaugt. Ausbeute 58 mg (53% d. Th.).

### Beispiel 20: 6-Chlor-7-((R)-3-hydroxypyrrolidin-1-yl)-2-piperazin-1-yl-4-pyrrolidin-1-yl-pteridin (Beispiel-Verbindung 49 aus Tabelle 1, nach Schema 3 hergestellt):

a) + b) 80 mg (0,26 mmol) 4-Pyrrolidin-1-yl-2,6,7-trichlor-pteridin werden in 5 ml Dioxan gelöst und mit 72 mg (0,58 mmol) (R)-3-Hydroxypyrrolidin und 100 µl (0,75 mmol) Diisopropylethylamin versetzt. Man rührt ca. 30 h bei 40° C. Das Reaktionsgemisch wird anschließend langsam zu einer 80° C heißen Lösung von 113 mg (1 mmol) Piperazin in 15 ml Dioxan getropft. Es wird ca. 16 h bei 80° C gerührt, auf Raumtemperatur abgekühlt und dann auf Eiswasser gegossen. Man extrahiert mit Dichlormethan, die organische Phase wird über Natriumsulfat getrocknet und im Vakuum vom Lösungsmittel befreit. Der Rückstand wird chromatographisch gereinigt. Ausbeute 68,8 mg (65% d. Th.).

### Beispiel 21: 6-Chlor-7-(3,4-dihydro-2H-1-benzopyran-4-yl)-oxy-2-piperazin-1-yl-4-pyrrolidin-1-yl-pteridin (Beispiel-Verbindung 88 aus Tabelle 1, nach Schema 2 hergestellt):

a) 2,6-Dichlor-7-(3,4-dihydro-2H-1-benzopyran-4-yl)-oxy-4-pyrrolidin-1-yl-pteridin: 100 mg (0,66 mmol) 4-Chromanol werden in 5 ml Tetrahydrofuran gelöst und bei -10° C unter Argon mit 330 µl (0,66 mmol) einer 2 molaren Lösung von Lithiumdiisopropylamid in Tetrahydrofuran versetzt. Man rührt 30 min bei Raumtemperatur, anschließend wird auf -10° C gekühlt und der Mischung langsam eine Lösung von 200 mg (0,66 mmol) 4-Pyrrolidin-1-yl-2,6,7-trichlor-pteridin in 10 ml Tetrahydrofuran zugetropft. Man rührt eine Stunde bei -10° C und lässt über Nacht auf Raumtemperatur kommen. Man versetzt mit Wasser und extrahiert das Reaktionsgemisch mit Dichlormethan. Die organische Phase wird über Natriumsulfat getrocknet und im Vakuum vom Lösungsmittel befreit. Der Rückstand wird chromatographisch gereinigt, Ausbeute 138 mg (50% d. Th.)
b) 6-Chlor-7-(3,4-dihydro-2H-1-benzopyran-4-yl)-oxy-2-piperazin-1-yl-4-pyrrolidin-1-yl-pteridin: Eine Lösung von 136 mg (0,33 mmol) 2,6-Dichlor-7-(3,4-dihydro-2H-1-benzopyran-4-yl)-oxy-4-pyrrolidin-1-yl-pteridin in 4 ml Dioxan wird zu einer 80° C heißen Lösung von 60 mg (0,7 mmol) Piperazin in 1 ml Dioxan getropft und ca. 16 h bei 80° C gerührt. Das Reaktionsgemisch wird im Vakuum vom Lösungsmittel befreit, der Rückstand wird chromatographisch gereinigt, Ausbeute 141 mg (93% d. Th.).

### Beispiel 22: 6-Chlor-2-piperazin-1-yl-4-pyrrolidin-1-yl-7-thiazolidin-3-yl-pteridin (Beispiel-Verbindung 100 aus Tabelle 1, nach Schema 3 hergestellt):

a) + b) 250 mg (0,82 mmol) 4-Pyrrolidin-1-yl-2,6,7-trichlor-pteridin werden in 10 ml Dioxan gelöst und mit 73 µl (1 mmol) Thiazolidin und 200 µl (1,1 mmol) Diisopropylethylamin versetzt. Man rührt ca. 16 h bei 40° C. Das Reaktionsgemisch wird anschließend langsam zu einer 80° C heißen Lösung von 354 mg (4 mmol) Piperazin in 15 ml Dioxan getropft. Es wird ca. 16 h bei 80° C gerührt, auf Raumtemperatur abgekühlt und dann auf Eiswasser gegossen. Man extrahiert mit Dichlormethan, die organische Phase wird über Natriumsulfat getrocknet und im Vakuum vom Lösungsmittel befreit. Der Rückstand wird wird chromatographisch gereinigt. Ausbeute 334 mg (54% d. Th.).

### Beispiel 23: 6-Chlor-7-(4-phenylpiperazin-1-yl)-2-piperazin-1-yl-4-pyrrolidin-1-yl-pteridin (Beispiel-Verbindung 102 aus Tabelle 1, nach Schema 3 hergestellt):

a) + b) 250 mg (0,82 mmol) 4-Pyrrolidin-1-yl-2,6,7-trichlor-pteridin werden in 10 ml Dioxan gelöst und mit 138 µl (1 mmol) 1-Phenylpiperazin und 200 µl (1,1 mmol) Diisopropylethylamin versetzt. Man rührt ca. 16 h bei 40° C. Das Reaktionsgemisch wird anschließend langsam zu einer 80° C heißen Lösung von 354 mg (4 mmol) Piperazin in 15 ml Dioxan getropft. Es wird ca. 5 h bei 80° C gerührt, auf Raumtemperatur abgekühlt und dann auf Eiswasser gegossen. Man extrahiert mit Dichlormethan, die organische Phase wird über Natriumsulfat getrocknet und im Vakuum vom Lösungsmittel befreit. Der Rückstand wird wird chromatographisch gereinigt. Ausbeute 332 mg (84% d. Th.).

### Beispiel 24: 6-Chlor-7-(3-(4-morpholinyl-methyl)-piperidin-1-yl)-2-piperazin-1-yl-4-pyrrolidin-1-yl-pteridin (Beispiel-Verbindung 106 aus Tabelle 1, nach Schema 3 hergestellt):

a) + b) 100 mg (0,33 mmol) 4-Pyrrolidin-1-yl-2,5,7-trichlor-pteridin werden in 7 ml Dioxan gelöst und mit 61 mg (0,33 mmol) 3-(4-Morpholinyl-methyl)-piperidin und 65 µl (0,49 mmol) Diisopropylethylamin versetzt. Man rührt ca. 16 h bei 40° C. Das Reaktionsgemisch wird anschließend langsam zu einer 80° C heißen Lösung von 141 mg (1,6 mmol) Piperazin in 15 ml Dioxan getropft. Es wird ca. 16 h bei 80° C gerührt, auf Raumtemperatur abgekühlt und dann auf Eiswasser gegossen. Man extrahiert mit Dichlormethan, die organische Phase wird über Natriumsulfat getrocknet und im Vakuum vom Lösungsmittel befreit. Der Rückstand wird mit etwas Dichlormethan aufgenommen und mit Petrolether versetzt, der entstandene Niederschlag wird abgesaugt. Ausbeute 80 mg (49% d. Th.).

### Beispiel 25: (S)-6-Chlor-7-(2-methyl-piperidin-1-yl)-2-piperazin-1-yl-4-pyrrolidin-1-yl-pteridin (Beispiel-Verbindung 107 aus Tabelle 1, nach Schema 3 hergestellt):

a) + b) 300 mg (0,99 mmol) 4-Pyrrolidin-1-yl-2,6,7-trichlor-pteridin werden in 15 ml Dioxan gelöst und mit 131 µl (1,08 mmol) (S)-(+)-2-Methylpiperidin und 182 µl (1,38 mmol) Diisopropylethylamin versetzt. Man rührt ca. 70 h bei 40° C. Das Reaktionsgemisch wird auf Raumtemperatur abgekühlt, mit Wasser versetzt und mit Dichlormethan extrahiert. Die organische Phase wird getrocknet und im Vakuum vom Lösungsmittel befreit. Man erhält 367 mg eines gelben Feststoffs. 345 mg dieses Feststoffs werden in 15 ml Dioxan gelöst und langsam zu einer 80° C heißen Lösung von 405 mg (4,7 mmol) Piperazin in 15 ml Dioxan getropft. Es wird ca. 16 h bei 80° C gerührt, auf Raumtemperatur abgekühlt und dann auf Eiswasser gegossen. Man extrahiert mit Dichlormethan, die organische Phase wird über Natriumsulfat getrocknet und im Vakuum vom Lösungsmittel befreit. Der Rückstand wird mit Ether versetzt, der entstandene Niederschlag wird abgesaugt. Ausbeute 280 mg (71% d. Th.).

### Beispiel 26: (R)-6-Chlor-7-(2-methyl-piperidin-1-yl)-2-piperazin-1-yl-4-pyrrolidin-1-yl-pteridin (Beispiel-Verbindung 108 aus Tabelle 1, nach Schema 3 hergestellt):

a) + b) 1,5 g (4,93 mmol) 4-Pyrrolidin-1-yl-2,6,7-trichlor-pteridin werden in 50 ml Dioxan gelöst und mit 653 µl (5 mmol) (R)-(+)-2-Methylpiperidin und 910 µl (6,9 mmol) Diisopropylethylamin versetzt. Man rührt ca. 70 h bei 40° C. Das Reaktionsgemisch wird auf Raumtemperatur abgekühlt, mit Wasser versetzt und mit Dichlormethan extrahiert. Die organische Phase wird getrocknet und im Vakuum vom Lösungsmittel befreit. Der Rückstand wird mit 40 ml Ether versetzt und für 15 Minuten gerührt. Der Niederschlag wird abgesaugt, mit Ether gewaschen und getrocknet. Man erhält 1,4 g eines Feststoffs, der in 60 ml Dioxan gelöst wird. Diese Lösung wird langsam zu einer 80° C heißen Lösung von 1,642 g (19 mmol) Piperazin in 60 ml Dioxan getropft. Es wird ca. 16 h bei 80° C gerührt, auf Raumtemperatur abgekühlt und dann auf Eiswasser gegossen. Man extrahiert mit Dichlormethan, die organische Phase wird über Natriumsulfat getrocknet und im Vakuum vom Lösungsmittel befreit. Der Rückstand wird chromatographisch gereinigt. Ausbeute 1,22 g (77% d. Th.).

### Beispiel 27: 6-Chlor-7-(3-hydroxymethyl-morpholin-4-yl)-2-piperazin-1-yl-4-pyrrolidin-1-yl-pteridin (Beispiel-Verbindung 111 aus Tabelle 1, nach Schema 3 hergestellt):

a) + b) 200 mg (0,66 mmol) 4-Pyrrolidin-1-yl-2,6,7-trichlor-pteridin werden in 10 ml Dioxan gelöst und mit 84,8 mg (0,72 mmol) 3-Hydroxymethylmorpholin und 121 µl (0,92 mmol) Diisopropylethylamin versetzt. Man rührt ca. 16 h bei 40° C. Das Reaktionsgemisch wird auf Raumtemperatur abgekühlt, mit Wasser versetzt und mit Dichlormethan extrahiert. Die organische Phase wird getrocknet und im Vakuum vom Lösungsmittel befreit. Der Rückstand wird chromatographisch gereinigt. 60 mg des so erhaltenen 7-(3-Hydroxymethyl-morpholin-4-yl)-2,6-dichlor-4-pyrrolidin-1-yl-pteridins werden in 10 ml Dioxan gelöst und langsam zu einer 80° C heißen Lösung von 67,3 mg (0,78 mmol) Piperazin in 10 ml Dioxan getropft. Es wird ca. 16 h bei 80° C gerührt, auf Raumtemperatur abgekühlt und dann auf Eiswasser gegossen. Man extrahiert mit Dichlormethan, die organische Phase wird über Natriumsulfat getrocknet und im Vakuum vom Lösungsmittel befreit. Der Rückstand wird chromatographisch gereinigt. Ausbeute 40 mg (59% d. Th.).

Alle Beispiel-Verbindungen aus Tabelle 1, die mit "Schema 3" gekennzeichnet sind, werden analog des Schemas 3 und analog der obigen ausführlichen Synthesevorschriften hergestellt.

Die nachfolgenden, nicht käuflichen Bausteine NR^{3.1}R^{3.2}, OR^{3.1} oder 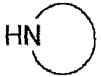 der Reaktionsschemata 1, 2 oder 3 wurden wie folgt synthetisiert:

Die Herstellung des für Beispiel 3 verwendeten (S)-2-(Pyrrolidin-2-yl)propan-2-ols ist beschrieben in: Enders, Dieter; Kipphardt, Helmut; Gerdes, Peter; Brena-Valle, Leonardo J.; Bhushan, Vidya., Bulletin des Societes Chimiques Belges (1988), 97(8-9), 691-704. Die Herstellung des für Beispiel 4 verwendeten (R)-2-(Pyrrolidin-2-yl)propan-2-ols erfolgt analog.

Die Herstellung des für Beispiel 90 der Tabelle verwendeten 4-Amino-1-tetrahydro-4H-pyran-4-yl-piperidins ist beschrieben in: Hoffmann, Matthias; Grauert, Matthias; Brandl, Trixi; Breitfelder, Steffen; Eickmeier, Christian; Steegmaier, Martin; Schnapp, Gisela; Baum, Anke; Quant, Jens Juergen; Solca, Flavio; Colbatzky, Florian. U.S. Pat. Appl. Publ. (2004), 109 pp., US 2004176380 A1.

Die Herstellung des für Beispiel 36 der Tabelle verwendeten 1-Amino-1-hydroxymethyl-cyclohexans ist beschrieben in: Meinzer, Alexandra; Breckel, Andrea; Thaher, Bassam Abu; Manicone, Nico; Otto, Hans-Hartwig. Helvetica Chimica Acta (2004), 87(1), 90-105.

Die Herstellung des für Beispiel 5 der Tabelle verwendeten (3R,4R)-Pyrrolidin-3,4-diols sowie des für Beispiel 6 verwendeten (3S,4S)-Pyrrolidin-3,4-diols ist beschrieben in: Lysek, Robert; Vogel, Pierre. Helvetica Chimica Acta (2004), 87(12), 3167-3181.

Das für Beispiel 77 der Tabelle verwendete 3-Phenyl-cyclopentylamin wird wie im Folgenden beschrieben hergestellt:
a) 3-Phenylcyclopentanon-oxim: 2.07 g ((29,8 mmol) Hydroxylamin werden in 4ml Wasser und 7 ml Methanol gelöst. Dazu tropft man eine Lösung von 2,2 g (13,7 mmol) 3-Phenylpentanon in 50 ml Methanol. Man gibt 1,89 g (13,7 mmol) Kaliumcarbonat und 9 ml Wasser zu und führt 2,5 h bei Raumtemperatur. Das Reaktionsgemisch wird im Vakuum vom Lösungsmittel befreit, der Rückstand wird in 130 ml Essigester aufgenommen. Die organische Phase wird mit 2 mal 50 ml Wasser gewaschen, die wässrigen Phasen werden mit 50 ml Essigester gegengewaschen. Die vereinigten organischen Phasen werden über Natriumsulfat getrocknet und im Vakuum vom Lösungsmittel befreit. Ausbeute 2,26 g (94% d. Th.).
b) 3-Phenyl-cyclopentylamin: 2,2 g (12,6 mmol) 3-Phenylcyclopentanon-oxim werden in 23 ml Methanol mit 4 ml 24%iger Ammoniaklösung und 2,2 g Raney-Nickel versetzt und bei Raumtemperatur mit 60 psi Wasserstoffdruck bis zum Ende der Wasserstoffaufnahme hydriert. Anschließend wird erneut 1 g Katalysator zugegeben und es wird wieder bis zum Ende der Wasserstoffaufnahme hydriert. Der Katalysator wird abfiltriert und das Lösungsmittel wird im Vakuum entfernt. Ausbeute 1,73 g (86% d. Th.).

Die Synthese von 4-Pyrrolidinyl-2,6,7-trichlorpteridin, welches als Ausgangsprodukt für die Synthese-Schemata 1, 2 und 3 dient, ist beschrieben in Merz, K.-H.; Marko, D.; Regiert, T.; Reiss, G.; Frank, W.; Eisenbrand, G. J. Med. Chem. (1998), 41, 4733-4743

Die Synthese von Tetrachlorpteridin, welches als Ausgangsprodukt für die Synthese von z.B. 4-Pyrrolidinyl-2,6,7-trichlorpteridin dient, ist beschrieben in: Schöpf, C.; Reichert, R.; Riefstahl, K. Liebigs Ann. Chem. (1941), 548, 82 - 94.

Das für die Beispiele 54 - 56 der Tabelle verwendete 4-Thiomorpholin-4-yl-2,6,7-trichlorpteridin wird wie folgt hergestellt:
10 g (37 mmol) Tetrachlorpteridin werden in 190 ml Chloroform gelöst und mit einer Lösung von 6,22 g (74 mmol) Natriumhydrogencarbonat in 70 ml Wasser versetzt. Die Mischung wird gerührt und im Eisbad gekühlt. Man tropft langsam eine Lösung von 3,73 ml (37 mmol) Thiomorpholin in 20 ml Chloroform zu. Man rührt noch eine Stunde unter Eiskühlung weiter, dann wird auf Raumtemperatur aufgewärmt und die organische Phase abgetrennt. Ein entstandener Niederschlag wird bei der wässrigen Phase belassen. Die wässrige Phase wird weiter mit Wasser verdünnt und mit Chloroform extrahiert. Die vereinigten organischen Phasen werden mit wenig Wasser gewaschen, über Natriumsulfat getrocknet und im Vakuum vom Lösungsmittel befreit. Der Rückstand wird zweimal aus Essigester umkristallisiert. Ausbeute 5,5 g (44% d. Th.).

Das für Beispiel 109 der Tabelle benötigte (S)-2-Amino-2-cyclopropylethanol wird wie folgt hergestellt:
500 mg (4,3 mmol) L-Cyclopropylglycin werden in 5 ml Tetrahydrofuran gelöst. Die Lösung wird auf 0° C gekühlt und es werden vorsichtig 8,69 ml (8,69 mmol) einer 1 molaren Lösung von Lithiumaluminiumhydrid in Tetrahydrofuran zugegeben so dass die Temperatur der Reaktionsmischung 10° C nicht übersteigt. Man rührt die entstandene Suspension ca. 16 Stunden bei Raumtemperatur. Dann versetzt man vorsichtig mit wenig Wasser, gibt Celite® 545 zu und saugt über Celite® 545 ab. Man wäscht mit Tetrahydrofuran nach, das Filtrat wird im Vakuum vom Lösungsmittel befreit. Der Rückstand wird ohne weitere Reinigung für die den nächsten Syntheseschritt eingesetzt.

Die Synthese des für Beispiel 112 der Tabelle eingesetzten (R)-3-Hydroxytetrahydropyrans ist beschrieben in Brown, H. C. und Vara Prasad, J. V. N.; J. Am. Chem. Soc. (1986), 108, 2049 - 2054.

Das für die Beispiele 113, 114 und 115 der Tabelle benötigte 4-Azetidin-1-yl-2,6,7-trichlorpteridin wird wie folgt hergestellt:
2 g (7,4 mmol) Tetrachlorpteridin werden in ca. 150 ml Chloroform gelöst und mit einer Lösung von 1,25 g (14 mmol) Natriumhydrogencarbonat in 60 ml Wasser versetzt. Man kühlt auf 0° C, versetzt mit einer Lösung von 0,5 ml (7,4 mmol) Azetidin in ca. 50 ml Chloroform und rührt eine Stunde bei 0° C weiter. Dann wird die organische Phase abgetrennt, mit Wasser gewaschen, über Natriumsulfat getrocknet und im Vakuum vom Lösungsmittel befreit. Der Rückstand wird mit Ether verrieben und abgesaugt. Ausbeute 780 mg (36% d. Th.), die Substanz wird ohne weitere Reinigung für die weiteren Schritte eingesetzt.

In der folgenden Tabelle 1 sind beispielhaft Verbindungen zusammengefasst, die nach einem der oben gezeigten Synthesewege hergestellt werden können. Entweder ist der Schmelzpunkt (mₚ) in°C angegeben oder der jeweilige M+H- Wert für die massenspektroskopische Charakterisierung der jeweiligen Verbindung ist angegeben.

Diese Verbindungen sind als PDE4-Inhibitoren geeignet und besitzen IC₅₀-Werte kleiner oder gleich 1 µmol.

Zur weiteren Erläuterung der vorliegenden Erfindung wurden Verbindungen der Formel 1 hergestellt, 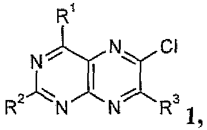 worin R¹, R² und R³ wie folgt definiert sind.

**Tabelle 1:**

| # | **R¹** | **R²** | **R³** | **Herstellverfahren** | **M+H oder mₚ** |
|---|---|---|---|---|---|
| 1 | 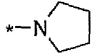 | 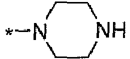 | 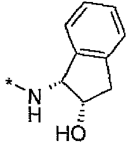 | Schema 1 | 467 / 469 |
| 2 | 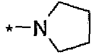 | 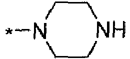 | 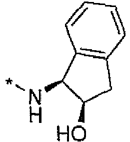 | Schema 1 | 467/469 |
| 3 | 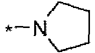 | 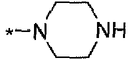 | 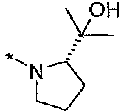 | Schema 3 | 447 / 449 |
| 4 | 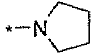 | 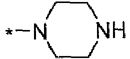 | 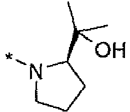 | Schema 3 | 447 / 449 |
| 5 | 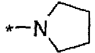 | 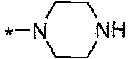 | 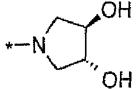 | Schema 3 | 421 / 423 |
| 6 | 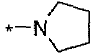 | 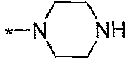 | 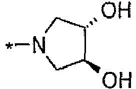 | Schema 3 | 421/423 |
| 7 | 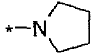 | 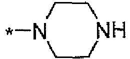 | 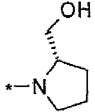 | Schema 3 | 407 / 409 |
| 8 | 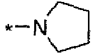 | 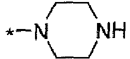 | 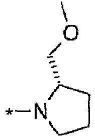 | Schema 3 | 433 / 435 |
| 9 | 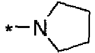 | 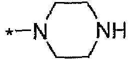 | 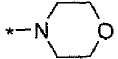 | Schema 3 | 405 / 407 |
| 10 | 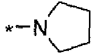 | 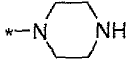 | 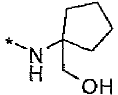 | Schema 1 | 485 / 487 |
| 11 | 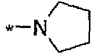 | 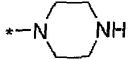 | 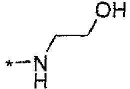 | Schema 1 | 378 / 381 |
| 12 | 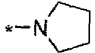 | 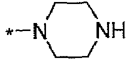 | 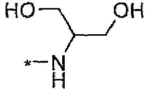 | Schema 1 | 409 / 411 |
| 13 | 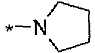 | 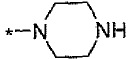 | 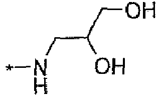 | Schema 1 | 409 / 411 |
| 14 | 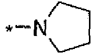 | 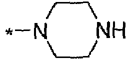 | 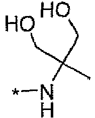 | Schema 1 | 423 / 425 |
| 15 | 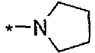 | 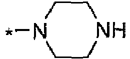 | 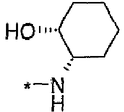 | Schema 1 | 433 / 435 |
| 16 | 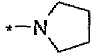 | 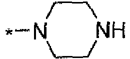 | 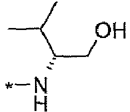 | Schema 1 | 421 / 423 |
| 17 | 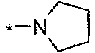 | 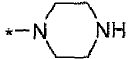 | 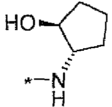 | Schema 1 | 419 / 421 |
| 18 | 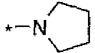 | 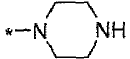 | 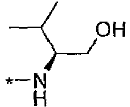 | Schema 1 | 421 / 423 |
| 19 | 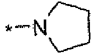 | 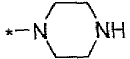 | 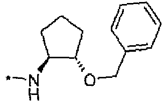 | Schema 1 | 509 / 511 |
| 20 | 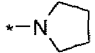 | 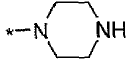 | 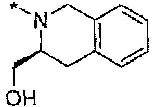 | Schema 3 | 481 / 483 |
| 21 | 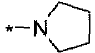 | 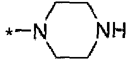 | 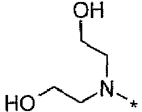 | Schema 1 | 423 / 425 |
| 22 | 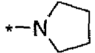 | 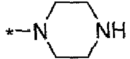 | 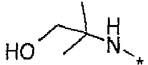 | Schema 1 | 407 / 409 |
| 23 | 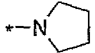 | 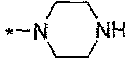 | 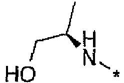 | Schema 1 | 393 / 395 |
| 24 | 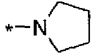 | 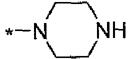 | 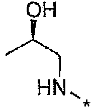 | Schema 1 | 393 / 395 |
| 25 | 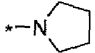 | 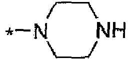 | 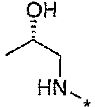 | Schema 1 | 393 / 395 |
| 26 | 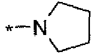 | 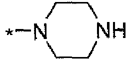 | 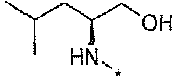 | Schema 1 | 435 / 437 |
| 27 | 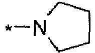 | 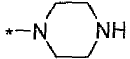 | 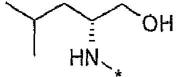 | Schema 1 | 435 / 437 |
| 28 | 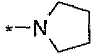 | 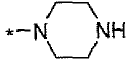 | 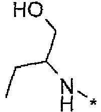 | Schema 1 | 407 / 409 |
| 29 | 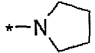 | 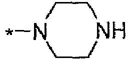 | 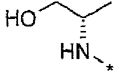 | Schema 1 | 393 / 395 |
| 30 | 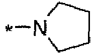 | 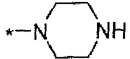 | 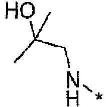 | Schema 1 | 407 / 409 |
| 31 | 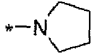 | 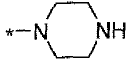 | 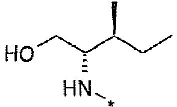 | Schema 1 | 435 / 437 |
| 32 | 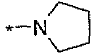 | 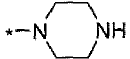 | 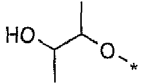 | Schema 2 | 408 / 410 |
| 33 | 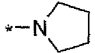 | 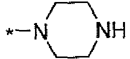 | 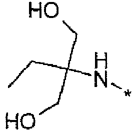 | Schema 1 | 437 /439 |
| 34 | 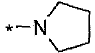 | 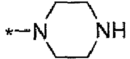 | 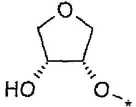 | Schema 2 | 422 / 424 |
| 35 | 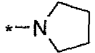 | 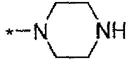 | 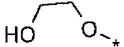 | Schema 2 | 380 / 382 |
| 36 | 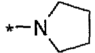 | 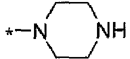 | 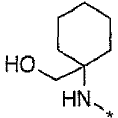 | Schema 1 | 447 / 449 |
| 37 | 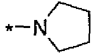 | 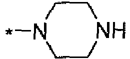 | 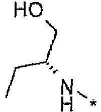 | Schema 1 | 407 / 409 |
| 38 | 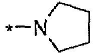 | 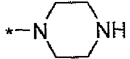 | 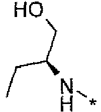 | Schema 1 | 407 / 409 |
| 39 | 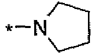 | 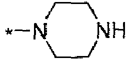 | 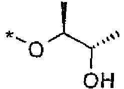 | Schema 2 | 408 / 410 |
| 40 | 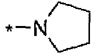 | 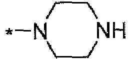 | 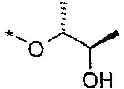 | Schema 2 | 408 / 410 |
| 41 | 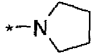 | 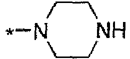 | 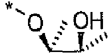 | Schema 2 | 408 / 410 |
| 42 | 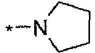 | 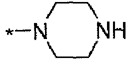 | 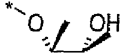 | Schema 2 | 408 / 410 |
| 43 | 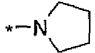 | 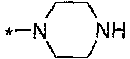 | 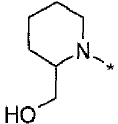 | Schema 3 | 433 / 435 |
| 44 | 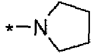 | 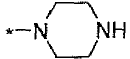 | 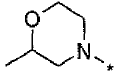 | Schema 3 | 419 / 421 |
| 45 | 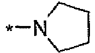 | 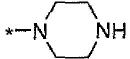 | 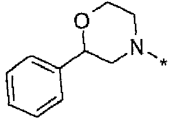 | Schema 3 | 481 / 483 |
| 46 | 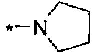 | 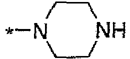 | 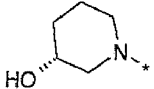 | Schema 3 | 419 / 421 |
| 47 | 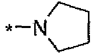 | 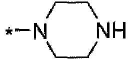 | 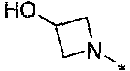 | Schema 3 | 391 / 393 |
| 48 | 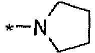 | 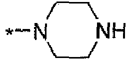 | 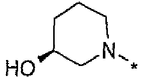 | Schema 3 | 419 / 421 |
| 49 | 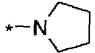 | 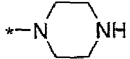 | 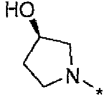 | Schema 3 | 405 / 407 |
| 50 | 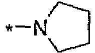 | 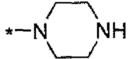 | 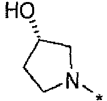 | Schema 3 | 405 / 407 |
| 51 | 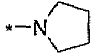 | 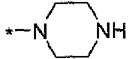 | 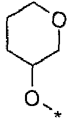 | Schema 2 | 420 / 422 |
| 52 | 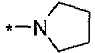 | 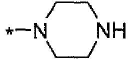 | 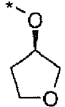 | Schema 2 | 406 / 408 |
| 53 | 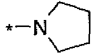 | 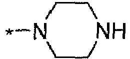 | 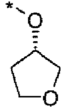 | Schema 2 | 406 / 408 |
| 54 | 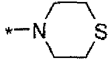 | 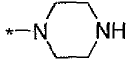 | 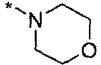 | Schema 3 | 437 / 439 |
| 55 | 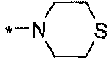 | 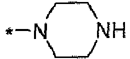 | 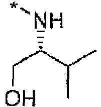 | Schema 1 | 453 / 455 |
| 56 | 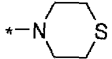 | 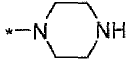 | 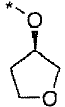 | Schema 2 | 438 / 440 |
| 57 | 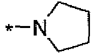 | 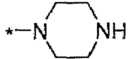 | 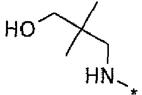 | Schema 1 | 421 / 423 |
| 58 | 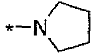 | 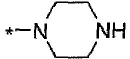 | 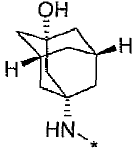 | Schema 1 | 485 / 487 |
| 59 | 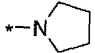 | 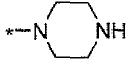 | 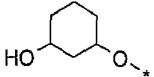 | Schema 2 | 434 / 436 |
| 60 | 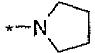 | 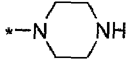 | 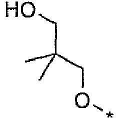 | Schema 2 | 422 / 424 |
| 61 | 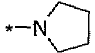 | 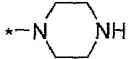 | 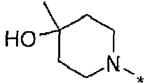 | Schema 3 | 433 / 435 |
| 62 | 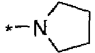 | 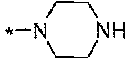 | 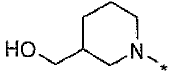 | Schema 3 | 433 / 435 |
| 63 | 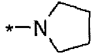 | 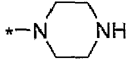 | 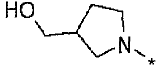 | Schema 3 | 419 / 421 |
| 64 | 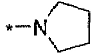 | 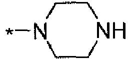 | 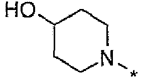 | Schema 3 | 419 / 421 |
| 65 | 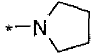 | 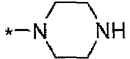 | 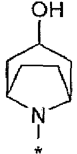 | Schema 3 | 445 / 447 |
| 66 | 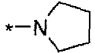 | 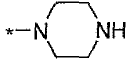 | 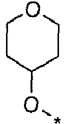 | Schema 2 | 420 / 422 |
| 67 | 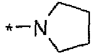 | 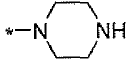 | 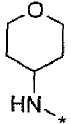 | Schema 1 | 419 / 421 |
| 68 | 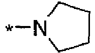 | 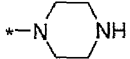 | 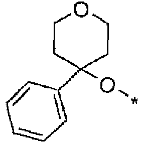 | Schema 2 | 496 / 498 |
| 69 | 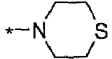 | 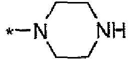 | 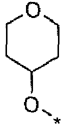 | Schema 2 | 452 / 454 |
| 70 | 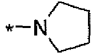 | 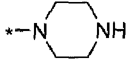 | 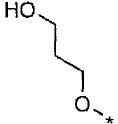 | Schema 2 | 394 / 396 |
| 71 | 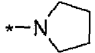 | 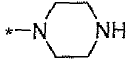 | 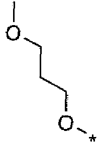 | Schema 2 | 408 / 410 |
| 72 | 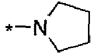 | 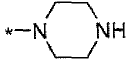 | 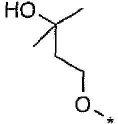 | Schema 2 | 422 / 424 |
| 73 | 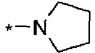 | 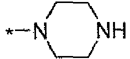 | 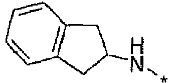 | Schema 1 | 451 / 453 |
| 74 | 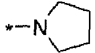 | 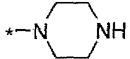 | 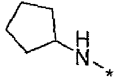 | Schema 1 | 403 / 405 |
| 75 | 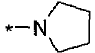 | 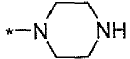 | 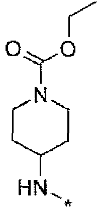 | Schema 1 | 490 / 492 |
| 76 | 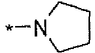 | 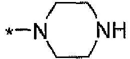 | 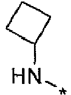 | Schema 1 | 389 / 391 |
| 77 | 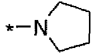 | 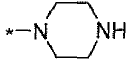 | 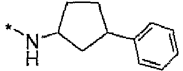 | Schema 1 | 479 / 481 |
| 78 | 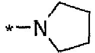 | 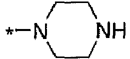 | 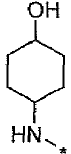 | Schema 1 | 433 / 435 |
| 79 | 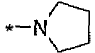 | 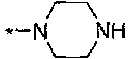 | 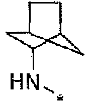 | Schema 1 | 429 / 431 |
| 80 | 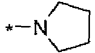 | 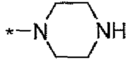 | 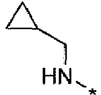 | Schema 1 | 389 / 391 |
| 81 | 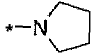 | 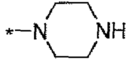 | 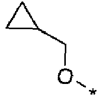 | Schema 2 | 390 / 392 |
| 82 | 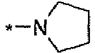 | 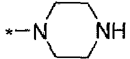 | 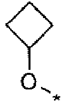 | Schema 2 | 390 / 392 |
| 83 | 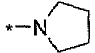 | 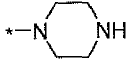 | 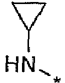 | Schema 1 | 375 / 377 |
| 84 | 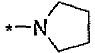 | 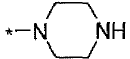 | 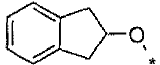 | Schema 2 | mₚ. 213-215° C |
| 85 | 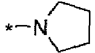 | 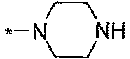 | 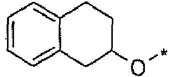 | Schema 2 | mₚ. 187-189° C |
| 86 | 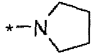 | 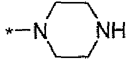 | 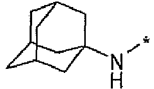 | Schema 1 | 469 / 471 |
| 87 | 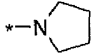 | 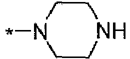 | 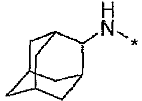 | Schema 1 | 469 / 471 |
| 88 | 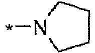 | 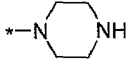 | 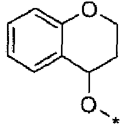 | Schema 2 | 468 / 470 |
| 89 | 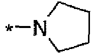 | 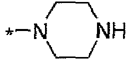 | 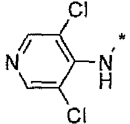 | Schema 1 | 480 / 482 / 484 / 486 |
| 90 | 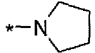 | 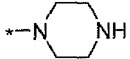 | 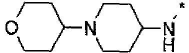 | Schema 1 | 502 / 504 |
| 91 | 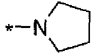 | 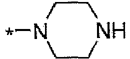 | 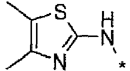 | Schema 1 | 446 / 448 |
| 92 | 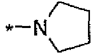 | 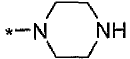 | 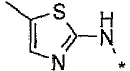 | Schema 1 | 432 / 434 |
| 93 | 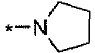 | 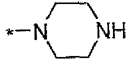 | 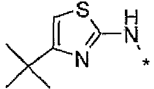 | Schema 1 | 474 / 476 |
| 94 | 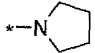 | 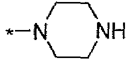 | 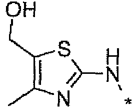 | Schema 1 | 476 / 478 |
| 95 | 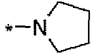 | 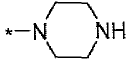 | 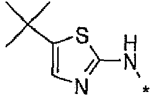 | Schema 1 | 474 / 476 |
| 96 | 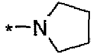 | 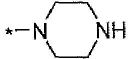 | 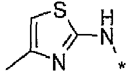 | Schema 1 | 432 / 434 |
| 97 | 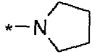 | 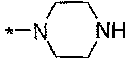 | 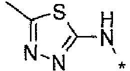 | Schema 1 | 433 / 435 |
| 98 | 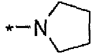 | 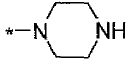 | 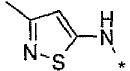 | Schema 1 | 432 / 434 |
| 99 | 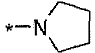 | 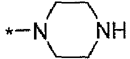 | *-NH₂ | Schema 1 | 335 / 337 |
| 100 | 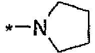 | 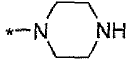 | 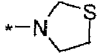 | Schema 3 | 407 / 409 |
| 101 | 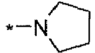 | 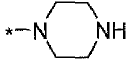 | 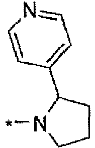 | Schema 3 | 446 / 448 |
| 102 | 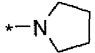 | 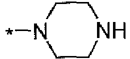 | 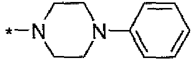 | Schema 3 | 480 / 482 |
| 103 | 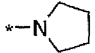 | 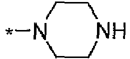 | 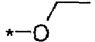 | Schema 2 | 364/ 366 178 - 180°C |
| 104 | 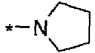 | 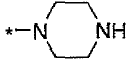 | 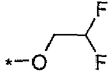 | Schema 2 | 400/402 193 - 195°C |
| 105 | 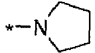 | 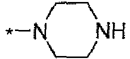 | *-OH | Schema 2 | 336 / 338 |
| 106 | 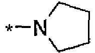 | 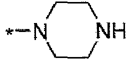 | 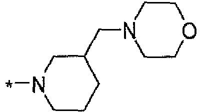 | Schema 3 | 502 / 504 |
| 107 | 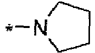 | 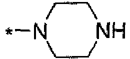 | 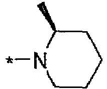 | Schema 3 | 417 / 419 |
| 108 | 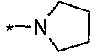 | 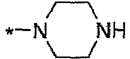 | 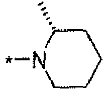 | Schema 3 | 417 / 419 |
| 109 | 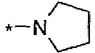 | 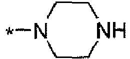 | 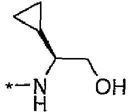 | Schema 1 | 419 / 421 |
| 110 | 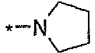 | 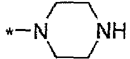 | 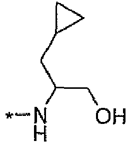 | Schema 1 | 433 / 435 |
| 111 | 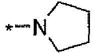 | 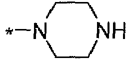 | 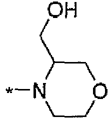 | Schema 3 | 435 / 437 |
| 112 | 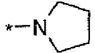 | 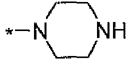 | 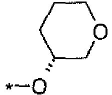 | Schema 2 | 420 / 422 |
| 113 | 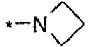 | 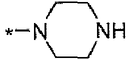 | 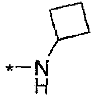 | Schema 3 | 375 / 377 |
| 114 | 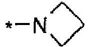 | 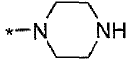 | 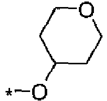 | Schema 2 | 406 / 408 |
| 115 | 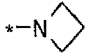 | 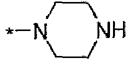 | 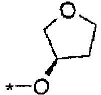 | Schema 2 | 392 / 394 |
| 116 | 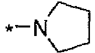 | 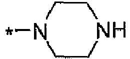 | 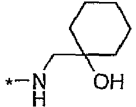 | Schema 1 | 447 / 449 |
| 117 | 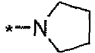 | 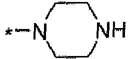 | 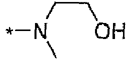 | Schema 1 | 393 / 395 |

### INDIKATIONSGEBIETE

Wie gefunden wurde, zeichnen sich die Verbindungen der Formel 1 durch vielfältige Anwendungsmöglichkeiten auf therapeutischem Gebiet aus. Hervorzuheben sind solche Anwendungsmöglichkeiten, für welche die erfindungsgemäßen Verbindungen der Formel 1 aufgrund ihrer pharmazeutischen Wirksamkeit als PDE4-Inhibitor bevorzugt zur Anwendung gelangen können. Beispielhaft genannt seien Atemwegs- oder gastrointestinale Erkrankungen oder Beschwerden, entzündliche Erkrankungen der Gelenke, der Haut oder der Augen, Krebserkrankungen, sowie Erkrankungen des periphären oder zentralen Nervensystems.

Hierbei bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einher gehen. Beispielhaft hierfür seien genannt, akute, allergische oder chronische Bronchitis, chronisch obstruktive Bronchitis (COPD), Husten, Lungenemphysem, allergische oder nicht-allergische Rhinitis oder Sinusitis, chronische Rhinitis oder Sinusitis, Asthma, Alveolitis, Farmers' Krankheit, hyperreaktive Atemwege, infektiöse Bronchitis oder Pneumonitis, pediatrisches Asthma, Bronchiektasien, Lungenfibrose, ARDS (akutes Atemnotsyndrom des Erwachsenen), Bronchialödem, Lungenödem, Bronchitis, Pneumonie oder interstitielle Pneumonie ausgelöst durch verschiedene Ursachen wie Aspiration, Inhalation von toxischen Gasen oder Bronchitis, Pneumonie oder interstitielle Pneumonie ausgelöst durch Herzinsuffizienz, Bestrahlung, Chemotherapie Lungenfibrose oder Mukoviszidose, alpha1-Antitrypsin-Mangel.

Ebenfalls bevorzugt genannt sei die Behandlung von entzündlichen Erkrankungen des Gastrointestinaltraktes. Beispielhaft hierfür seien genannt, akute oder chronische entzündliche Veränderungen bei Gallenblasenentzündung, Morbus Crohn, Colitis ulcerosa, entzündliche Pseudopolypen, juvenile Polypen, Colitis cystica profunda, pneumatosis cystoides interstinales, Erkrankungen der Gallengänge und Gallenblase, z.B. Gallensteine und Konglomerate, zur Behandlung von entzündlichen Erkrankungen der Gelenke wie rheumatoide Arthritis oder entzündliche Erkrankungen der Haut und der Augen.

Ebenfalls bevorzugt genannt sei die Behandlung von Krebserkrankungen. Beispielhaft hierfür seien genannt alle Formen von akuten und chronischen Leukämien wie akute lymphatische Leukämie (ALL), akute myeloische Leukämie (AML), akute nicht lymphozytische Leukämie (ANLL), chronisch lymphatische Leukämie (CLL), chronisch myeloische Leukämie (CML), Haar-Zell-Leukämie, akute promyelocytische Leukämie (APL), insbesondere die APL-Subform mit einer chromosomalen t(15; 17)-Translokation, Erkrankungen der lymphatischen Organe, Hodgkin-Lymphome und non-Hodgkin Lymphome, sowie Knochentumoren wie das Osteosarkom und sowie alle Arten von Gliomen wie Oligodendrogliom und Glioblastom.

Des Weiteren bevorzugt genannt sei die Vorbeugung und Behandlung von Erkrankungen des periphären oder zentralen Nervensystems. Beispielhaft hierfür seien genannt Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Besonders bevorzugt betrifft die vorliegende Erfindung die Verwendung von Verbindungen der Formel 1 zur Herstellung eines Arzneimittels zur Behandlung entzündlicher oder obstruktiver Erkrankungen der oberen und unteren Atmungsorgane einschließlich der Lunge wie beispielsweise allergische Rhinitis, chronische Rhinitis, Bronchiectasis, zystische Fibrose, idiopathische Lungenfibrose, fibrosierende Alveolitis, COPD, chronische Bronchitis, chronischer Sinusitis, Asthma, insbesondere COPD, chronische Bronchitis und Asthma.

Am meisten bevorzugt ist die Verwendung der Verbindungen der Formel **1** zur Behandlung von entzündlichen und obstruktiven Erkrankungen wie COPD, chronische Bronchitis, chronischer Sinusitis, Asthma, Morbus Crohn, Colitis ulcerosa, insbesondere COPD, chronische Bronchitis und Asthma.

Ebenfalls bevorzugt ist die Verwendung der Verbindungen der Formel **1** zur Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Ein herausragender Aspekt der vorliegenden Erfindung ist das reduzierte Profil an Nebenwirkungen. Darunter wird im Rahmen der Erfindung verstanden, eine Dosis einer pharmazeutischen Zusammensetzung verabreichen zu können, ohne beim Patienten Erbrechen, bevorzugt Übelkeit, besonders bevorzugt Unwohlsein auszulösen. Höchst bevorzugt ist die Verabreichung einer therapeutisch wirksamen Substanzmenge, ohne Emesis oder Nausea auszulösen, in jedem Stadium des Krankheitsverlaufs.

### DARREICHUNGSFORMEN

Einen weiteren Aspekt der Erfindung bilden Medikamente zur Behandlung von Atemwegserkrankungen, die eines oder mehrere der oben genannten Pteridine der Formel **1** enthalten.

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0,1 bis 90 Gew.-%, bevorzugt 0,5 bis 50 Gew.-% der Gesamtzusammensetzung liegen, d.h. in Mengen die ausreichend sind, um den unten angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel **1** gemäß der obigen bevorzugten Ausführungsformen.

Besonders bevorzugt ist es, wenn die Verbindungen der Formel **1** oral verabreicht werden, besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie *p*-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnussoder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmack-Aufbesserern oder Farbstoffen versetzt werden.

Ebenfalls bevorzugt ist es, wenn die Verbindungen der Formel **1** inhalativ verabreicht werden, besonders bevorzugt ist es, wenn die Verabreichung ein oder zweimal täglich erfolgt. Hierzu müssen die Verbindungen der Formel **1** in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhaltionslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### Inhalationspulver

Sind die Verbindungen der Formel **1** im Gemisch mit physiologisch unbedenklichen Hilfsstoffen enthalten, können zur Darstellung der erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Verfahren zur Herstellung der erfindungsgemäßen Inhalationspulver durch Mahlen und Mikronsieren sowie durch abschließendes Mischen der Bestandteile sind aus dem Stand der Technik bekannt.

### Treibgashaltige Inhalationsaerosole

Die im Rahmen der erfindungsgemäßen Verwendung einsetzbaren treibgashaltigen Inhalationsaerosole können **1** im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind fluorierte Alkanderivate ausgewählt aus TG134a (1,1,1,2-Tetrafluorethan), TG227 (1,1,1,2,3,3,3-Heptafluorpropan) und Mischungen derselben. Die im Rahmen der erfindungsgemäßen Verwendung einsetzbaren treibgashaltigen Inhalationsaerosole können ferner weitere Bestandteile wie Co-Solventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

### Treibgasfreie Inhalationslösungen

Die erfindungsgemäße Verwendung von Verbindungen der Formel **1** erfolgt bevorzugt zur Herstellung von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Die Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfingdungsgemäß besonders bevorzugt wird Salzsäure zur Einstellung des pH-Werts verwendet.

Den im Rahmen der erfindungsgemäßen Verwendung einsetzbaren treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.
Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration.

Für die oben beschriebenen Behandlungsformen werden gebrauchsfertige Packungen eines Medikaments zur Behandlung von Atemwegserkrankungen, beinhaltend eine beigelegte Beschreibung, welche beispielsweise die Worte Atemwegserkrankung, COPD oder Asthma enthalten, und ein Pteridin der Formel **1** bereit gestellt.

## Patentansprüche

1. Verbindungen der Formel 1, 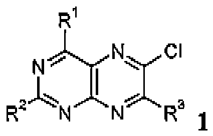 worin
**R¹** ein Rest ausgewählt aus der Gruppe bestehend aus einem gesättigten oder teilweise gesättigten vier-, fünf-, sechs- oder siebengliedrigen Heterocyclus und ein fünf- oder sechsgliedriger Heteroaromat, der ein Stickstoffatom enthält und gegebenenfalls ein weiteres Atom ausgewählt aus der Gruppe bestehend aus Stickstoff, Schwefel und Sauerstoff enthalten kann;
und
**R²** ein Rest ausgewählt aus der Gruppe bestehend aus einem gesättigten oder teilweise gesättigten fünf-, sechs- oder siebengliedrigen Heterocyclus und einem fünf- oder sechsgliedrigen Heteroaromat, der ein Stickstoffatom enthält und gegebenenfalls ein weiteres Atom ausgewählt aus der Gruppe bestehend aus Stickstoff, Schwefel und Sauerstoff enthalten kann;
und worin
**R³** NR^{3.1}R³.² oder OR^{3.1}, wobei
**R^{3.1}** und **R^{3.2}** jeweils unabhängig voneinander
H oder ein Rest ausgewählt aus der Gruppe bestehend aus C₁₋₆-Alkyl, ein- oder mehrwertiges, verzweigtes oder unverzweigtes C₁₋₆-Alkanol, C₁₋₆-Halogenalkyl, C₁₋₆-alkylen-O-C₁₋₂-Alkyl, ein mono- oder bicyclisches, gesättigtes oder teilweise gesättigtes C₃₋₁₀-Cycloalkyl-, mono- oder bicyclischer, gesättigter oder teilweise gesättigter, vier- bis zehngliedriger Heterocyclus mit 1 bis 3 Heteroatomen ausgewählt aus S, N oder O, und einem mono- oder bicyclischen, fünf- bis zehngliedrigen Heteroaromat, mit 1 bis 4 Heteroatomen ausgewählt aus S, N oder O,
der gegebenenfalls mit einem oder mehreren Resten ausgewählt aus der Gruppe bestehend aus OH, Halogen, C₁₋₆-Alkyl, C₁₋₆-Alkanol, C₁₋₆-Haloalkyl, COOR^{3.3}, O-C₁₋₆-Alkyl, C₆₋₁₀-Aryl, C₃₋₁₀-Cycloalkyl, vier- bis zehngliedriger Heterocyclus, fünfbis zehngliedriger Heteroaromat und O-C₁₋₄-Alkyl-Phenyl substituiert sein kann,
wobei dieser Rest gegebenenfalls wiederum mit mindestens einem Rest ausgewählt aus der Gruppe bestehend aus Halogen, OH, C₁₋₃-Alkyl, C₁₋₃-Halogenalkyl substituiert sein kann,
und worin
**R^{3.3}** H, C₁₋₆-Alkyl oder (C₁₋₆-Alkanol),
oder worin
**R³** ein gesättigter oder teilweise gesättigter vier-, fünf-, sechs- oder siebengliedriger Heterocyclus, der ein Stickstoffatom enthält und gegebenenfalls ein oder zwei weitere Atome ausgewählt aus der Gruppe bestehend aus Stickstoff, Schwefel und Sauerstoff enthalten kann und
der gegebenenfalls mit einem oder mehreren Resten ausgewählt aus der Gruppe bestehend aus OH, Halogen, C₁₋₆-Alkyl, C₁₋₆-Alkanol, C₁₋₄-Alkylen-O-C₁₋₃-Alkyl, C₆₋₁₀Aryl, C₃₋₁₀-Cycloalkyl, fünf- bis zehngliedriges Heteroaryl, vier- bis zehngliedriger Heterocyclus" der ein Stickstoffatom enthält und gegebenenfalls 1 oder 2 weitere Heteroatome ausgewählt aus N, S oder O enthalten kann, C₁₋₂-alkylen-C₅₋₁₀-Heteroaryl und C₁₋₂-alkylen-C₄₋₁₀-Heterocyclus, der ein Stickstoffatom enthält und gegebenenfalls 1 oder 2 weitere Heteroatome ausgewählt aus N, S oder O enthalten kann, substituiert sein kann,
welcher wiederum gegebenenfalls durch einen oder mehrere Reste ausgewählt aus der Gruppe bestehend aus Methyl, Ethyl, O-Methyl, Cl, F und OH substituiert sein kann,
oder worin
**R³** ein gesättigter oder teilweise gesättigter, bi- oder polycyclischer sieben-, acht, neun- oder zehngliedriger Heterocyclus, der ein Stickstoffatom enthält und der gegebenenfalls ein, zwei oder drei weitere Atome ausgewählt aus der Gruppe bestehend aus Stickstoff, Schwefel und Sauerstoff enthalten kann und der gegebenenfalls mit einem oder mehreren Resten ausgewählt aus der Gruppe bestehend aus OH, (Halogen), C₁₋₆-Alkyl, C₁₋₆-Alkanol, C₁₋₄-Alkyl-O-C₁₋₃-Alkyl, C₆₋₁₀-Aryl, C₃₋₁₀-Cycloalkyl, fünf- bis zehngliedriges Heteroaryl, vier- bis zehngliedriger Heterocyclus, C₁₋₂-alkylen-C₅₋₁₀-Heteroaryl und C₁₋₂-alkylen-C₄₋₁₀-Heterocyclus substituiert sein kann,
welcher wiederum gegebenenfalls durch einen oder mehrere Reste ausgewählt aus der Gruppe bestehend aus Methyl, Ethyl, O-Methyl, Cl, F und OH substituiert sein kann,
bedeutet, sowie pharmakologisch verträgliche Salze, Diastereomere, Enantiomere, Racemate, Hydrate oder Solvate davon.

2. Verbindungen der Formel **1** mach Anspruch 1, worin
**R¹** ein gesättigter oder ungesättigter vier-, fünf-oder sechsgliedriger Heterocyclus oder Heteroaromat, der ein Stickstoffatom enthält und gegebenenfalls ein weiteres Atom ausgewählt aus der Gruppe bestehend aus Stickstoff, Schwefel und Sauerstoff enthalten kann;
und
**R²** ein fünf-, sechs- oder siebengliedriger Heterocyclus oder Heteroaromat, der ein Stickstoffatom enthält und gegebenenfalls ein weiteres Atom ausgewählt aus der Gruppe bestehend aus Stickstoff, Schwefel und Sauerstoff enthalten kann;
und worin
**R³** NR^{3.1}R^{3.2} oder OR^{3.1}, wobei
**R^{3.1}** und **R^{3.2}** jeweils unabhängig voneinander
H oder ein Rest ausgewählt aus der Gruppe bestehend aus C₁₋₆-Alkyl, ein- oder mehrwertiges, verzweigtes oder unverzweigtes C₁₋₆-Alkanol, C₁₆₆-Halogenalkyl, C₁₋₆-alkylen-O-C₁₋₂-Alkyl, mono- oder bicyclisches, gesättigtes oder teilweise gesättigtes C₃₋₁₀-Cycloalkyl, , mono- oder bicyclischer, gesättigter oder teilweise gesättigter, vier- bis zehngliedriger Heterocyclus mit 1 oder 2 Heteroatomen ausgewählt aus S, N oder O, und einem mono- oder bicyclischen, fünf- bis zehngliedrigen Heteroaromat, mit 1, 2 oder 3 Heteroatomen ausgewählt aus S, N oder O,
der gegebenenfalls mit einem oder mehreren Resten ausgewählt aus der Gruppe bestehend aus OH, C₁₋₆-Alkyl, C₁₋₆-Alkanol, COO-C₁₋₃-Alkyl, O-C₁₋₃-Alkyl, Phenyl, C₃₋₁₀-Cycloalkyl, vier- bis zehngliedriger Heterocyclus, fünf- bis zehngliedriger Heteroaromat, und O-CH₂-Phenyl substituiert sein kann,
wobei dieser Rest gegebenenfalls wiederum mit mindestens einem Rest ausgewählt aus der Gruppe bestehend aus Halogen, OH, C₁₋₃-Alkyl und C₁₋₃-Halogenalkyl substituiert sein kann,
oder worin
**R³** ein gesättigter oder teilweise gesättigter vier-, fünf-, sechs- oder siebengliedriger Heterocyclus, der ein Stickstoffatom enthält und gegebenenfalls ein oder zwei weitere Atome ausgewählt aus der Gruppe bestehend aus Stickstoff, Schwefel und Sauerstoff enthalten kann und
der gegebenenfalls mit einem oder mehreren Resten ausgewählt aus der Gruppe bestehend aus OH, C₁₋₆-Alkyl, C₁₋₆-Alkanol, CH₂-O-CH₃, Phenyl, C₃₋₁₀-Cycloalkyl, fünf- bis zehngliedriges Heteroaryl, vier- bis zehngliedriger Heterocyclus, der ein Stickstoffatom enthält und gegebenenfalls 1 oder 2 weitere Heteroatome ausgewählt aus N, S oder O enthalten kann, CH₂-C₅₋₁₀-Heteroaryl und CH₂-C₄₋₁₀-Heterocyclus, der ein Stickstoffatom enthält und gegebenenfalls 1 oder 2 weitere Heteroatome ausgewählt aus N, S oder O enthalten kann, substituiert sein kann, welcher wiederum gegebenenfalls durch einen oder mehrere Reste ausgewählt aus der Gruppe bestehend aus Methyl, O-Methyl, Cl und OH substituiert sein kann,
oder worin
**R³** ein gesättigter oder teilweise gesättigter, bi- oder polycyclischer sieben-, acht, neun- oder zehngliedriger Heterocyclus, der ein Stickstoffatom enthält und der gegebenenfalls ein, zwei oder drei weitere Atome ausgewählt aus der Gruppe bestehend aus Stickstoff, Schwefel und Sauerstoff enthalten kann und der gegebenenfalls mit einem oder mehreren Resten ausgewählt aus der Gruppe bestehend aus OH, C₁₋₆-Alkyl, C₁₋₆-Alkanol, CH₂-O-CH₃, Phenyl, C₃₋₁₀-Cycloalkyl, fünf- bis zehngliedriger Heteroaryl, vier- bis zehngliedriger HeterocyclusCH₂-C₅₋₁₀-Heteroaryl und CH₂-C₄₋₁₀-Heterocyclus substituiert sein kann,
welcher wiederum gegebenenfalls durch einen oder mehrere Reste ausgewählt aus der Gruppe bestehend aus Methyl, O-Methyl, Cl und OH substituiert sein kann,
bedeutet, sowie pharmakologisch verträgliche Salze, Diastereomere, Enantiomere, Racemate, Hydrate oder Solvate davon.

3. Verbindungen der Formel **1** nach Anspruch 1, worin
**R³** ein gesättigter oder teilweise gesättigter vier-, fünf-, sechs- oder siebengliedriger Heterocyclus, der ein Stickstoffatom enthält und über dieses Stickstoffatom mit dem Rest des Moleküls verknüpft ist und der gegebenenfalls ein oder zwei weitere Atome ausgewählt aus der Gruppe bestehend aus Stickstoff, Schwefel und Sauerstoff enthalten kann und
der gegebenenfalls mit einem oder mehreren Resten ausgewählt aus der Gruppe bestehend aus OH, (Halogen), C₁₋₆-Alkyl, C₁₋₆-Alkanol, C₁₋₄-Alkyl-O-C₁₋₃-Alkyl, C₆₋₁₀-Aryl, C₃₋₁₀-Cycloalkyl, fünf- bis zehngliedriges Heteroaryl, vier- bis zehngliedriger Heterocyclus, , der ein Stickstoffatom enthält und gegebenenfalls 1 oder 2 weitere Heteroatome ausgewählt aus N, S oder O enthalten kann, C₁₋₂-alkylen-C₅₋₁₀-Heteroaryl und C₁₋₂-alkylen-C₄₋₁₀-Heterocyclus, der ein Stickstoffatom enthält und gegebenenfalls 1 oder 2 weitere Heteroatome ausgewählt aus N, S oder O enthalten kann, substituiert sein kann,
welcher wiederum gegebenenfalls durch einen oder mehrere Reste ausgewählt aus der Gruppe bestehend aus Methyl, Ethyl, O-Methyl, Cl, F und OH substituiert sein kann,
oder worin
**R³** ein gesättigter oder teilweise gesättigter, bi- oder polycyclischer sieben-, acht, neun- oder zehngliedriger Heterocyclus, der ein Stickstoffatom enthält und über dieses Stickstoffatom mit dem Rest des Moleküls verknüpft ist und der gegebenenfalls ein, zwei oder drei weitere Atome ausgewählt aus der Gruppe bestehend aus Stickstoff, Schwefel und Sauerstoff enthalten kann und der gegebenenfalls mit einem oder mehreren Resten ausgewählt aus der Gruppe bestehend aus OH, (Halogen), C₁₋₆-Alkyl, C₁₋₆-Alkanol, C₁₋₄-Alkyl-O-C₁₋₃-Alkyl, C₆₋₁₀-Aryl, C₃₋₁₀-Cycloalkyl, fünf- bis zehngliedriges Heteroaryl, vier- bis zehngliedriger Heterocyclus, C₁₋₂-alkylen-C₅₋₁₀-Heteroaryl und C₁₋₂-alkylen-C₄₋₁₀-Heterocyclus substituiert sein kann,
welcher wiederum gegebenenfalls durch einen oder mehrere Reste ausgewählt aus der Gruppe bestehend aus Methyl, Ethyl, O-Methyl, Cl, F und OH substituiert sein kann,
bedeutet,
sowie pharmakologisch verträgliche Salze, Diastereomere, Enantiomere, Racemate, Hydrate oder Solvate davon.

4. Verbindungen der Formel **1** mach einem der Ansprüche 1 bis 3, worin
**R¹** Pyrrolidin oder Azetidin bedeutet, sowie pharmakologisch verträgliche Salze, Diastereomere, Enantiomere, Racemate, Hydrate oder Solvate davon.

5. Verbindungen der Formel **1** nach einem der Ansprüche 1 bis 3, worin
**R²** Piperazin bedeutet, sowie pharmakologisch verträgliche Salze, Diastereomere, Enantiomere, Racemate, Hydrate oder Solvate davon.

6. Verbindungen der Formel **1** nach einem der Ansprüche 1 bis 5, worin
**R³** NHR^{3.1} bedeutet,
sowie pharmakologisch verträgliche Salze, Diastereomere, Enantiomere, Racemate, Hydrate oder Solvate davon.

7. Verbindungen der Formel **1** mach Anspruch 6, worin
**R^{3.2}** ein verzweigtes oder unverzweigtes, ein- oder mehrwertiges C₁₋₆-Alkanol oder ein C₃₋₆-Cycloalkyl bedeutet sowie pharmakologisch verträgliche Salze, Diastereomere, Enantiomere, Racemate, Hydrate oder Solvate davon.

8. Verbindungen der Formel **1** nach einem der Ansprüche 1 bis 5 , worin
**R³** NHR^{3.1} oder OR^{3.1}
**R^{3.1}** ein gesättigter oder ungesättigter, fünf- oder sechsgliedriger Heterocyclus mit 1 oder 2 Heteroatomen unabhängig voneinander ausgewählt aus der Gruppe bestehend aus O, S und N,
der gegebenenfalls mit mindestens einem der Reste ausgewählt aus der Gruppe bestehend aus
OH, Methyl, Ethyl, einem verzweigten oder unverzweigten C₁₋₄-Alkanol, Phenyl und C₃₋₁₀-Cycloalkyl substituiert sein kann.

9. Verbindungen der Formel 1 nach Anspruch 8, worin
**R^{3.1}** Tetrahydrofuryl oder Tetrahydropyranyl ist, der gegebenenfalls mit mindestens einem der Reste ausgewählt aus der Gruppe bestehend aus
OH, Methyl, Ethyl, einem verzweigten oder unverzweigten C₁₋₄-Alkanol, Phenyl und C₃₋₁₀-Cycloalkyl substituiert sein kann.

10. Verbindungen der Formel **1** nach einem der Ansprüche 1 bis 5, worin
**R³** ein Rest ausgewählt aus der Gruppe bestehend aus einem gesättigten fünfoder sechsgliedrigen Heterocyclus und einem bicyclischen gesättigten oder teilweise gesättigten acht-, neun- oder zehngliedrigen Heterocyclus, der ein Stickstoffatom enthält und über dieses Stickstoffatom mit dem Rest des Moleküls verknüpft ist und der gegebenenfalls ein weiteres Atom ausgewählt aus der Gruppe bestehend aus Stickstoff, Schwefel und Sauerstoff enthalten kann und gegebenenfalls mit einem oder mehreren Resten ausgewählt aus der Gruppe bestehend aus OH, Methyl, Ethyl, einem verzweigten oder unverzweigten C₁₋₄-Alkanol, Phenyl, C₃₋₁₀-Cycloalkyl, einem fünf- bis zehngliedrigen Heteroaryl und einem vier- bis zehngliedrigen Heterocyclus substituiert sein kann,
bedeutet,
sowie pharmakologisch verträgliche Salze, Diastereomere, Enantiomere, Racemate, Hydrate oder Solvate davon.

11. Verbindungen der Formel 1 nach Anspruch 10, worin
**R³** Pyrrolidin, das über das Stickstoffatom mit dem restlichen Molekül verknüpft ist und das gegebenenfalls mit einem oder mehreren Resten ausgewählt aus der Gruppe bestehend aus aus OH, Methyl, Ethyl und einem verzweigten oder unverzweigten C₁₋₄-Alkanol substituiert sein kann,
bedeutet,
sowie pharmakologisch verträgliche Salze, Diastereomere, Enantiomere, Racemate, Hydrate oder Solvate davon.

12. Verbindungen der Formel 2 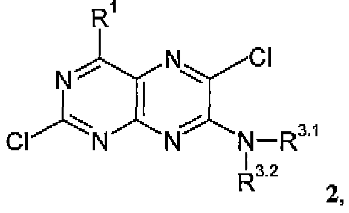 der Formel 3 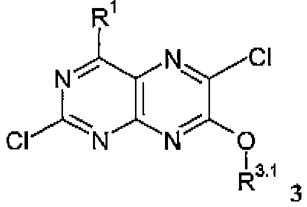 oder der Formel 4 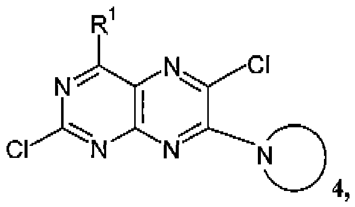 wobei R^{3.1} und R^{3.2} die in Anspruch 1 definierten Bedeutungen haben,
wobei R¹ die Bedeutungen Pyrrolidinyl, Azetidinyl oder Thiomorpholinyl haben und wobei 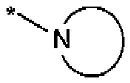 ausgewählt ist aus der Gruppe bestehend aus einem gesättigten oder teilweise gesättigten vier-, fünf-, sechs- oder siebengliedrigen monocyclischen Heterocyclus oder einem sieben- bis zehngliedrigen bicyclischen Heterocyclus, der über ein Stickstoffatom mit dem Rest des Moleküls verknüpft ist und dergegebenenfalls ein oder zwei weitere Atome ausgewählt aus der Gruppe bestehend aus Stickstoff, Schwefel und Sauerstoff enthalten kann und
der gegebenenfalls mit einem oder mehreren Resten ausgewählt aus der Gruppe bestehend aus OH, (Halogen), C₁₋₆-Alkyl, C₁₋₆-Alkanol, C₁₋₄-Alkyl-O-C₁₋₃-Alkyl, C₆₋₁₀-Aryl, C₃₋₁₀-Cycloalkyl, fünf- bis zehngliedriges Heteroaryl, vier- bis zehngliedriger Heterocyclus, C₁₋₂-alkylen-C₅₋₁₀-Heteroaryl und C₁₋₂-alkylen-C₄₋₁₀-Heterocyclus substituiert sein kann,
welcher wiederum gegebenenfalls durch einen oder mehrere Reste ausgewählt aus der Gruppe bestehend aus Methyl, Ethyl, O-Methyl, Cl, F und OH substituiert sein kann.

13. Verbindungen nach einem der Ansprüche 1 bis 11 als Arzneimittel.

14. Verwendung von Verbindungen nach einem der Ansprüche 1 bis 11 zur Herstellung eines Medikaments zur Behandlung von Atemwegs- oder gastrointestinalen Beschwerden oder Erkrankungen, wie auch entzündliche Erkrankungen der Gelenke, der Haut oder der Augen, Krebserkrankungen, sowie von Erkrankungen des periphären oder zentralen Nervensystems.

15. Verwendung von Verbindungen nach einem der Ansprüche 1 bis 11 zur Herstellung eines Medikaments zur Vorbeugung und Behandlung von Atemwegs- oder Lungenerkrankungen, die mit einer erhöhten Schleimproduktion, Entzündungen und / oder obstruktiven Erkrankungen der Atemwege einhergehen.

16. Verwendung von Verbindungen nach einem der Ansprüche 1 bis 11 zur Herstellung eines Medikament zur Behandlung von entzündlichen und obstruktiven Erkrankungen wie COPD, chronischer Sinusitis, Asthma, Morbus Crohn, Colitis ulcerosa.

17. Verwendung von Verbindungen nach einem der Ansprüche 1 bis 11 zur Herstellung eines Medikaments zur Vorbeugung und Behandlung von Erkrankungen des periphären oder zentralen Nervensystems wie Depression, bipolare oder manische Depression, akute und chronische Angstzustände, Schizophrenie, Alzheimer'sache Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Him-Trauma.

18. Verwendung von Verbindungen nach einem der Ansprüche 1 bis 11 zur Herstellung eines Medikaments zur Behandlung von Krebserkrankungen wie akuten und chronischen Leukämien, akute lymphatische und akute myeloische Leukämie, chronisch lymphatische und chronisch myeloische Leukämie, Erkrankungen der lymphatischen Organe, Hodgkin-Lymphome und non-Hodgkin Lymphome sowie Knochentumoren wie das Osteosarkom und sowie alle Arten von Gliomen wie Oligodendrogliom und Glioblastom.

## Claims

1. Compounds of formula 1, 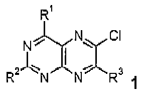 wherein
**R¹** denotes a group selected from among a saturated or partially saturated four-, five-, six- or seven-membered heterocycle and a five- or six-membered heteroaromatic group, which contains a nitrogen atom and may optionally contain another atom selected from among nitrogen, sulphur and oxygen;
and
**R²** denotes a group selected from among a saturated or partially saturated five-, six- or seven-membered heterocycle and a five- or six-membered heteroaromatic group, which contains a nitrogen atom and may optionally contain another atom selected from among nitrogen, sulphur and oxygen;
and wherein
**R³** denotes NR^{3.1}R^{3.2} or OR^{3.1}, wherein
**R^{3.1}** and **R^{3.2}** each independently of one another denote
H or a group selected from among C₁₋₆-alkyl, mono- or polyvalent, branched or unbranched C₁₋₆-alkanol, C₁₋₆-haloalkyl, C₁₋₆-alkylene-O-C₁₋₂-alkyl, a mono- or bicyclic, saturated or partially saturated C₃₋₁₀-cycloalkyl, mono- or bicyclic C₆₋₁₀-aryl, mono- or bicyclic, saturated or partially saturated, four- to ten-membered heterocycle with 1 to 3 heteroatoms selected from S, N or O, and a mono- or bicyclic, five- to ten-membered heteroaromatic group with 1 to 4 heteroatoms selected from S, N or O, which may optionally be substituted by one or more groups selected from among OH, (halogen), C₁₋₆-alkyl, C₁₋₆-alkanol, (C₁₋₆-haloalkyl), COOR^{3.3}, O-C₁₋₆-alkyl, C₆₋₁₀-aryl, C₃₋₁₀-cycloalkyl, four- to ten-membered heterocycle, five- to ten-membered heteroaromatic group and O-C₁₋₄-alkyl-phenyl, while this group may in turn optionelly be substituted by at least one group selected from among halogen, OH, C₁₋₃-alkyl, C₁₋₃-haloalkyl,
and wherein
**R^{3.3}** denotes H, C₁₋₆-alkyl or (C₁₋₆-alkanol),
or wherein
**R³** denotes a saturated or partially saturated four-, five-, six- or seven-membered heterocycle which contains a nitrogen atom and may optionally contain one or two other atoms selected from among nitrogen, sulphur and oxygen and
which may optionally be substituted by one or more groups selected from among OH, halogen, C₁₋₆-alkyl, C₁₋₆-alkanol, C₁₋₄-alkylene-O-C₁₋₃-alkyl, C₈₋₁₀-aryl, C₃₋₁₀-cycloalkyl, five- to ten-membered heteroaryl, four- to ten-membered heterocycle, which contains a nitrogen atom and may optionally contain 1 or 2 further heteroatoms selected from N, S or O, C₁₋₂-alkylone-C₅₋₁₀-heteroaryl and C₁₋₂-alkylene-C₄₋₁₀ heterocycle, which contains a nitrogen atom and may optionally contain 1 or 2 further heteroatoms selected from N, S or O,
which may in turn optionally be substituted by one or more groups selected from among methyl, ethyl, O-methyl, Cl, F and OH,
or wherein
**R³** denotes a saturated or partially saturated, bi- or polycyclic seven-, eight-, nine- or ten-membered heterocycle, which contains a nitrogen atom and may optionally contain one, two or three other atoms selected from among nitrogen, sulphur and oxygen and which may optionally be substituted by one or more groups selected from among OH, halogen, C₁₋₆-alkyl, C₁₋₆-alkanol, C₁₋₄-alkyl-O-C₁₋₃-alkyl, C₆₋₁₀-aryl, C₃₋₁₀-cycloalkyl, five-to ten-membered heteroaryl, four-to ten-membered heterocycle, C₁₋₂-alkylene-C₅₋₁₀-heteroaryl and C₁₋₂-alkylene-C₄₋₁₀ heterocycle,
which may in turn optionally be substituted by one or more groups selected from among methyl, ethyl, O-methyl, Cl, F and OH,
and the pharmacologically acceptable salts, diastereomers, enantiomers, racemates, hydrates or solvates thereof.

2. Compounds of formula **1** according to claim 1, wherein
**R¹** denotes a saturated or unsaturated four, five- or six-membered heterocycle or heteroaromatic group, which contains a nitrogen atom and may optionally contain another atom selected from among nitrogen, sulphur and oxygen;
and
**R²** denotes a five-, six- or seven-membered heterocycle or heteroaromatic group which contains a nitrogen atom and may optionally contain another atom selected from among nitrogen, sulphur and oxygen;
and wherein
**R³** denotes NR^{3.1}R^{3.2} or OR^{3.1}, wherein
**R^{3.1}** and **R^{3.2}** each independently of one another denote
H or a group selected from among C₁₋₆-alkyl, mono- or polyvalent, branched or unbranched C₁₋₆-alkanol, C₁₋₆-haloalkyl, C₁₋₆-alkylene-O-C₁₋₂-alkyl, mono- or bicyclic, saturated or partially saturated C₃₋₁₀-cycloalkyl, mono- or bicyclic, saturated or partially saturated, four- to ten-membered heterocycle with 1 or 2 heteroatoms selected from S, N or O, and a mono-or bicyclic, five- to ten-membered heteroaromatic group with 1, 2 or 3 heteroatoms selected from S, N or O, which may optionally be substituted by one or more groups selected from among OH, C₁₋₆-alkyl, C₁₋₆-alkanol, COO-C₁₋₃-alkyl, O-C₁₋₃-alkyl, phenyl, C₃₋₁₀-cycloalkyl, four- to ten-membered heterocycle, five- to ten-membered heteroaromatic group and O-CH₂-phenyl,
while this group may in turn optionally be substituted by at least one group selected from among halogen, OH, C₁₋₃-alkyl and C₁₋₃-haloalkyl,
or wherein
**R³** denotes a saturated or partially saturated four-, five-, six- or seven-membered heterocycle, which contains a nitrogen atom and may optionally contain one or two other atoms selected from among nitrogen, sulphur and oxygen and
which may optionally be substituted by one or more groups selected from among OH, C₁₋₆-alkyl, C₁₋₆-alkanol, CH₂-O-CH₃, phenyl, C₃₋₁₀-cycloalkyl, five- to ten-membered heteroaryl, four- to ten-membered heterocycle, which contains a nitrogen atom and may optionally contain 1 or 2 further heteroatoms selected from N, S or O, CH₂-C₅₋₁₀-heteroaryl and CH₂-C₄₋₁₀ heterocycle, which contains a nitrogen atom and may optionally contain 1 or 2 further heteroatoms selected from N, S or O,
which may in turn optionally be substituted by one or more groups selected from among methyl, O-methyl, Cl and OH,
or wherein
**R³** denotes a saturated or partially saturated , bi- or polycyclic seven-, eight-, nine- or ten-membered heterocycle, which contains a nitrogen atom and which may optionally contain one, two or three other atoms selected from among nitrogen, sulphur and oxygen and
which may optionally be substituted by one or more groups selected from among OH, C₁₋₆-alkyl, C₁₋₆-alkanol, CH₂-O-CH₃, phenyl, C₃₋₁₀-cycloalkyl, five-to ten-membered heteroaryl, four-to ten-membered heterocycle, CH₂-C₅₋₁₀-heteroaryl and CH₂-C₄₋₁₀ heterocycle,
which may in turn optionally be substituted by one or more groups selected from among methyl, O-methyl, Cl and OH,
and the pharmacologically acceptable salts, diastereomers, enantiomers, racemates, hydrates or solvates thereof.

3. Compounds of formula 1 according to claim 1, wherein
**R³** denotes a saturated or partially saturated four-, five-, six- or seven-membered heterocycle which contains a nitrogen atom and is linked to the rest of the molecule via this nitrogen atom and which may optionally contain one or two other atoms selected from among nitrogen, sulphur and oxygen and
which may optionally be substituted by one or more groups selected from among OH, (halogen), C₁₋₆-alkyl, C₁₋₆-alkanol, C₁₋₄-alkyl-O-C₁₋₃-alkyl, C₆₋₁₀-aryl, C₃₋₁₀-cycloalkyl, five-to ten-membered heteroaryl, four-to ten-membered heterocycle, which contains a nitrogen atom and may optionally contain 1 or 2 further heteroatoms selected from N, S or O, C₁₋₂-alkylene-C₅₋₁₀-heteroaryl and C₁₋₂-alkylene-C₄₋₁₀ heterocycle, which contains a nitrogen atom and may optionally contain 1 or 2 further heteroatoms selected from N, S or 0, which may in turn optionally be substituted by one or more groups selected from among methyl, ethyl, O-methyl, Cl, F and OH,
or wherein
**R³** denotes a saturated or partially saturated, bi- or polycyclic seven-, eight-, nine- or ten-membered heterocycle, which contains a nitrogen atom and is linked to the rest of the molecule via this nitrogen atom and which may optionally contain one, two or three other atoms selected from among nitrogen, sulphur and oxygen and which may optionally be substituted by one or more groups selected from among OH, (halogen), C₁₋₆-alkyl, C₁₋₆-alkanol, C₁₋₄-alkyl-O-C₁₋₃-alkyl, C₆₋₁₀-aryl, C₃₋₁₀-cycloalkyl, five- to ten-membered heteroaryl, four- to ten-membered heterocycle, C₁₋₂-alkylene-C₅₋₁₀-heteroaryl and C₁₋₂-alkylene-C₄₋₁₀ heterocycle, which may in turn optionally be substituted by one or more groups selected from among methyl, ethyl, O-methyl, Cl, F and OH,
and pharmacologically acceptable salts, diastereomers, enantiomers, racemates, hydrates or solvates thereof.

4. Compounds of formula **1** according to one of claims 1 to 3, wherein
**R¹** denotes pyrrolidine or azetidine,
and pharmacologically acceptable salts, diastereomers, enantiomers, racemates, hydrates or solvates thereof.

5. Compounds of formula **1** according to one of claims 1 to 3, wherein
**R²** denotes piperazine,
and pharmacologically acceptable salts, diastereomers, enantiomers, racemates, hydrates or solvates thereof.

6. Compounds of formula **1** according to one of claims 1 to 5, wherein
**R³** denotes NHR^{3.1},
and pharmacologically acceptable salts, diastereomers, enantiomers, racemates, hydrates or solvates thereof.

7. Compounds of formula **1** according to claim 6, wherein
**R^{3.2}** denotes a branched or unbranched, mono- or polyvalent C₁₋₆-alkanol or a C₃₋₆-cycloalkyl,
and phermecologicelly acceptable salts, diastereomers, enantiomers, racemates, hydrates or solvates thereof.

8. Compounds of formula **1** according to one of claims 1 to 5 , wherein
**R³** denotes NHR^{3.1} or OR^{3.1}
**R^{3.1}** denotes a saturated or unsaturated, five- or six-membered heterocycle with 1 or 2 heteroatoms selected independently of one another from among O, S and N,
which may optionally be substituted by at least one of the groups selected from among OH, methyl, ethyl, a branched or unbranched C₁₋₄-alkanol, phenyl and C₅₋₁₀-cycloalkyl.

9. Compounds of formula 1 according to claim 8, wherein
**R^{3.1}** is tetrahydrofuryl or tetrahydropyranyl, which may optionally be substituted by at least one of the groups selected from among OH, methyl, ethyl, a branched or unbranched C₁₋₄-alkanol, phenyl and C₃₋₁₀-cycloalkyl.

10. Compounds of formula **1** according to one of claims 1 to 5, wherein
**R³** denotes a group selected from among a saturated five- or six-membered heterocycle and a bicyclic saturated or partially saturated eight-, nine- or ten-membered heterocycle which contains a nitrogen atom and is linked to the rest of the molecule via this nitrogen atom and which may optionally contain another atom selected from among nitrogen, sulphur and oxygen and which may optionally be substituted by one or more groups selected from among OH, methyl, ethyl, a branched or unbranched C₁₋₄-alkanol, phenyl, C₃₋₁₀-cycloalkyl, a five- to ten-membered heteroaryl and a four- to ten-membered heterocycle,
as well as pharmacologically acceptable salts, diastereomers, enantiomers, racemates, hydrates or solvates thereof.

11. Compounds of formula 1 according to claim 10, wherein
**R³** denotes pyrrolidine, which is linked to the rest of the molecule via the nitrogen atom and which may optionally be substituted by one or more groups selected from among OH, methyl, ethyl and a branched or unbranched C₁₋₄-alkanol,
and pharmacologically acceptable salts, diastereomers, enantiomers, racemates, hydrates or solvates thereof.

12. Compounds of formula **2** 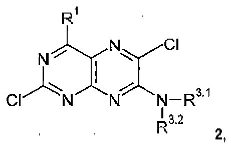 of formula 3 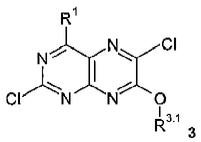 or of formula 4 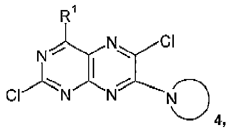 wherein R^{3.1} and R^{3.2} have the meanings defined in claim 1,
wherein R¹ have the meanings pyrrolidinyl, azetidinyl or thiomorpholinyl and wherein 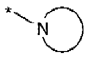 is selected from among a saturated or partially saturated four-, five-, six-or seven-membered monocyclic heterocycle or a seven- to ten-membered bicyclic heterocycle, which is linked to the rest of the molecule via a nitrogen atom and which may optionally contain one or two other atoms selected from among nitrogen, sulphur and oxygen and which may optionally be substituted by one or more groups selected from among OH, (halogen), C₁₋₆-alkyl, C₁₋₆-alkanol, C₁₋₄-alkyl-O-C₁₋₃-alkyl, C₆₋₁₀-aryl, C₃₋₁₀-cycloalkyl, five- to ten-membered heteroaryl, four- to ten-membered heterocycle, C₁₋₂-alkylene-C₅₋₁₀-heteroaryl and C₁₋₂-alkylene-C₄₋₁₀ heterocycle,
which may in turn optionally be substituted by one or more groups selected from among methyl, ethyl, O-methyl, Cl, F and OH.

13. Compounds according to one of claims 1 to 11 as medicaments.

14. Use of compounds according to one of claims 1 to 11 for preparing a medicament for the treatment of respiratory or gastrointestinal complaints or diseases, such as inflammatory diseases of the joints, skin or eyes, cancers, and diseases of the peripheral or central nervous system.

15. Use of compounds according to one of claims 1 to 11 for preparing a medicament for the prevention and treatment of respiratory or pulmonary diseases which are accompanied by increased mucus production, inflammations and/or obstructive diseases of the respiratory tract.

16. Use of compounds according to one of claims 1 to 11 for preparing a medicament for the treatment of inflammatory and obstructive diseases such as COPD, chronic sinusitis, asthma, Crohn's disease and ulcerative colitis.

17. Use of compounds according to one of claims 1 to 11 for preparing a medicament for the prevention and treatment of diseases of the peripheral or central nervous system such as depression, bipolar or manic depression, acute and chronic anxiety states, schizophrenia, Alzheimer's disease, Parkinson's disease, acute and chronic multiple sclerosis or acute and chronic pain and brain damage caused by stroke, hypoxia or cranio-cerebral trauma.

18. Use of compounds according to one of claims 1 to 11 for preparing a medicament for the treatment of cancers such as acute and chronic leukaemias, acute lymphatic and acute myeloid leukaemia, chronic lymphatic and chronic myeloid leukaemia, diseases of the lymphatic organs, Hodgkin's lymphomas and non-Hodgkin's lymphomas and bone tumours such as osteosarcoma and all kinds of gliomas such as oligodendroglioma and glioblastoma.

## Revendications

1. Composés de formule 1 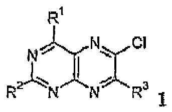 dans laquelle
R¹ représente un radical choisi dans le groupe comprenant un hétérocycle de quatre, cinq, six ou sept chaînons, saturé ou partiellement saturé et un composé hétéro-aromatique de cinq ou six chaînons qui contient un atome d'azote et qui peut contenir éventuellement un autre atome choisi dans le groupe comprenant l'azote, le soufre et l'oxygène ;
et
R² représente un radical choisi dans le groupe comprenant un hétérocycle de cinq, six ou sept chaînons, saturé ou partiellement saturé et un composé hétéro-aromatique de cinq ou six chaînons qui contient un atome d'azote et qui peut contenir éventuellement un autre atome choisi dans le groupe comprenant l'azote, le souffre et l'oxygène ;
et dans laquelle
R³ représente NR^{3.1}R^{3.2} ou OR^{3.1}, dans laquelle
R^{3.1} et R^{3.2} représentent respectivement, indépendamment l'un de l'autre
H ou un radical choisi dans le groupe comprenant un groupe alkyle en C₁ à C₆, alcanol en C₁ à C₆ mono- ou multivalent, ramifié ou non ramifié, halogénoalkyle en C₁ à C₆, alkylène en C₁ à C₆-O-alkyle en C₁ à C₂, un groupe cycloalkyle en C₃ à C₁₀, mono- ou bicyclique, saturé ou partiellement saturé, un hétérocycle de quatre à dix chaînons mono-ou bicyclique, saturé ou partiellement saturé, comportant 1 à 3 hétéroatomes choisis parmi S, N ou O et un composé hétéro-aromatique de cinq à dix chaînons, mono- ou bicyclique comportant 1 à 4 hétéroatomes choisis parmi S, N ou O,
qui peut être substitué éventuellement par un ou plusieurs radicaux choisis dans le groupe comprenant OH, un atome d'halogène, un groupe alkyle en C₁ à C₆, alcanol en C₁ à C₆, halogéno-alkyle en C₁ à C₆, COOR^{3.3}, O-alkyle en C₁ à C₆, aryle en C₆ à C₁₀, cycloalkyle en C₃ à C₁₀, un hétérocycle de quatre à dix chaînons, un composé hétéro-aromatique de cinq à dix chaînons et un groupe O-alkyle en C₁ à C₄
dans laquelle ce radical peut être substitué éventuellement à nouveau par au moins un radical choisi dans le groupe comprenant un atome d'halogène, OH, un groupe alkyle en C₁ à C₃, halogénoalkyle en C₁ à C₃,
et dans laquelle
R^{3.3} représente H, un groupe alkyle en C₁ à C₆ ou (alcanol en C₁ à C₆),
ou dans laquelle
R³ représente un hétérocycle de quatre, cinq, six ou sept chaînons, saturé ou partiellement saturé, qui peut contenir un atome d'azote et éventuellement un ou deux autres atomes choisis dans le groupe comprenant un atome d'azote, de soufre et d'oxygène et
qui peut éventuellement être substitué par un ou plusieurs radicaux choisis dans le groupe comprenant OH, un atome d'halogène, un groupe alkyle en C₁ à C₆, alcanol en C₁ à C₆, alkylène en C₁ à C₄-O-alkyle en C₁ à C₃, aryle en C₆ à C₁₀, cycloalkyle en C₃ à C₁₀, un groupe hétéroaryle de cinq à dix chaînons, un hétérocycle de quatre à dix chaînons qui peut contenir un atome d'azote et éventuellement 1 ou 2 autres hétéroatomes choisis parmi N, S ou O,un groupe alkylène en C₁ à C₂-hétéroaryle en C₅ à C₁₀ et alkylène en C₁ à C₂-hétérocycle en C₄ à C₁₀ qui contient un atome d'azote et peut contenir éventuellement 1 ou 2 autres hétéroatomes choisis parmi N, S ou O,
qui peut éventuellement être substitué à nouveau par un ou plusieurs radicaux choisis dans le groupe comprenant un groupe méthyle, éthyle, 0-méthyle, Cl, F et OH
ou dans laquelle
R³ représente un hétérocycle de sept, huit, neuf ou dix chaînons, saturé ou partiellement saturé, bi- ou polycyclique, qui contient un atome d'azote et qui peut contenir éventuellement un, deux ou trois autres atomes choisis dans le groupe comprenant un atome d'azote, de soufre et d'oxygène et qui peut être substitué éventuellement par un ou plusieurs radicaux choisis dans le groupe comprenant OH, (un atome d'halogène), un groupe alkyle en C₁ à C₆, alcanol en C₁ à C₆, alkyle en C₁ à C₄-O-alkyle en C₁ à C₃, aryle en C₆ à C₁₀, cycloalkyle en C₃ à C₁₀, un hétéroaryle de cinq à dix chaînons, un hétérocycle de quatre à dix chaînons, un groupe alkylène en C₁ à C₂-hétéro-aryle en C₅ à C₁₀ et alkylène en C₁ à C₂-hétérocycle en C₄ à C₁₀, qui peut éventuellement être substitué à nouveau,
par un ou plusieurs radicaux choisis dans le groupe comprenant un groupe méthyle, éthyle, 0-méthyle, Cl, F et OH,
et leurs sels, diastéréomères, énantiomères, racémates, hydrates ou solvates pharmaceutiquement acceptables.

2. Composés de formule 1 selon la revendication 1, dans laquelle
R¹ représente un hétérocycle ou un composé hétéro-aromatique de quatre, cinq ou six chaînons saturé ou insaturé, qui contient un atome d'azote et qui peut contenir éventuellement un autre atome choisi dans le groupe comprenant l'azote, le soufre et l'oxygène ;
et
R² représente un hétérocycle ou un composé hétéro-aromatique de cinq, six ou sept chaînons, qui contient un atome d'azote et qui peut contenir éventuellement un autre atome choisi dans le groupe comprenant l'azote, le souffre et l'oxygène ;
et dans laquelle
R³ représente NR^{3.1}R^{3.2} ou OR^{3.1}, dans laquelle
R^{3.1} et R^{3.2} représentent respectivement, indépendamment l'un de l'autre
H ou un radical choisi dans le groupe comprenant un groupe alkyle en C₁ à C₆, alcanol en C₁ à C₆ mono- ou multivalent, ramifié, ou non ramifié, halogénoalkyle en C₁ à C₆, alkylène en C₁ à C₆-O-alkyle en C₁ à C₂, un groupe cycloalkyle en C₃ à C₁₀, mono- ou bicyclique, saturé ou partiellement saturé, un hétérocycle de quatre à dix chaînons mono- ou bicyclique, saturé ou partiellement saturé, comportant 1 à 2 hétéroatomes choisis parmi S, N ou O et un composé hétéro-aromatique de cinq à dix chaînons, mono- ou bicyclique comportant 1, 2 ou 3 hétéroatomes choisis parmi S, N ou O,
qui peut être substitué éventuellement par un ou plusieurs radicaux choisis dans le groupe comprenant OH, un groupé alkyle en C₁ à C₆, alcanol en C₁ à C₆, COO-alkyle en C1 à C₃, O-alkyle en C₁ à C₃, phényle, cycloalkyle en C₃ à C₁₀, un hétérocycle de quatre à dix chaînons, un composé hétéro-aromatique de cinq à dix chaînons et un groupe O-CH₂-phényle,
dans laquelle ce radical peut être substitué éventuellement à nouveau par au moins un radical choisi dans le groupe comprenant un atome d'halogène, OH, un groupe alkyle en C₁ à C₃ et halogéno-alkyle en C₁ à C₃,
ou dans laquelle
R³ représente un hétérocycle de quatre, cinq, six ou sept chaînons, saturé ou partiellement saturé, qui contient un atome d'azote et peut contenir éventuellement un ou deux autres atomes choisis dans le groupe comprenant un atome d'azote, de soufre et d'oxygène et
qui peut être substitué éventuellement par un ou plusieurs radicaux choisis dans le groupe comprenant OH, un groupe alkyle en C₁ à C₆, alcanol en C₁ à C₆, CH₂-O-CH₃, phényle, cycloalkyle en C₃ à C₁₀, un groupe hétéroaryle de cinq à dix chaînons, un hétérocycle de quatre à dix chaînons qui contient un atome d'azote et peut contenir éventuellement 1 ou 2 autres hétéroatomes choisis parmi N, S ou O, un groupe CH₂-hétéroaryle en C₅ à C₁₀ et CH₂-hétéroaryle en C₄ à C₁₀ qui contient un atome d'azote et peut contenir éventuellement 1 ou 2 autres hétéroatomes choisis parmi N, S ou O,
qui peut éventuellement être substitué à nouveau
par un ou plusieurs radicaux choisis dans le groupe comprenant un groupe méthyle, O-méthyle, Cl et OH,
ou dans laquelle
R³ représente un hétérocycle de sept, huit, neuf ou dix chaînons, saturé ou partiellement saturé, bi- ou polycyclique, qui contient un atome d'azote et qui peut contenir éventuellement un, deux ou trois autres atomes choisis dans le groupe comprenant un atome d'azote, de soufre et d'oxygène et qui peut être substitué éventuellement par un ou plusieurs radicaux choisis dans le groupe comprenant OH, un groupe alkyle en C₁ à C₆, alcanol en C₁ à C₆, CH₂-O-CH₃, phényle, cycloalkyle en C₃ à C₁₀, un hétéroaryle de cinq à dix chaînons, un hétérocycle de quatre à dix chaînons-CH₂-hétéro-aryle en C₅ à C₁₀ et CH₂-hétérocycle en C₄ à C₁₀, qui peut éventuellement être substitué à nouveau par un ou plusieurs radicaux choisis dans le groupe comprenant un groupe méthyle, 0-méthyle, Cl et OH,
et leurs sels, diastéréomères, énantiomères, racémates, hydrates ou solvates pharmaceutiquement acceptables.

3. Composés de formule 1 selon la revendication 1, dans laquelle
R³ représente un hétérocycle de quatre, cinq, six ou sept hétérocycles saturé ou partiellement saturé, qui contient un atome d'azote et est relié par cet atome d'azote, au radical de la molécule et qui peut contenir éventuellement un ou deux autres atomes choisis dans le groupe comprenant un atome d'azote, de soufre et d'oxygène et
qui peut être substitué éventuellement par un ou plusieurs radicaux choisis dans le groupe comprenant OH, (un atome d'halogène), un groupe alkyle en C₁ à C₆, alcanol en C₁ à C₆, alkyle en C₁ à C₄-O-alkyle en C₁ à C₃, aryle en C₆ à C₁₀, cycloalkyle en C₃ à C₁₀, un groupe hétéroaryle de cinq à dix chaînons, un hétérocycle de quatre à dix chaînons qui contient un atome d'azote et peut contenir éventuellement 1 ou 2 autres hétéroatomes choisis parmi N, S ou O,un groupe alkylène en C₁ à C₂-hétéroaryle en C₅ à C₁₀ et alkylène en C₁ à C₂-hétérocycle en C₄ à C₁₀ qui contient un atome d'azote et peut contenir éventuellement 1 ou 2 autres hétéroatomes choisis parmi N, S ou 0,
qui peut éventuellement être substitué à nouveau par un ou plusieurs radicaux choisis dans le groupe comprenant un groupe méthyle, éthyle, O-méthyle, Cl, F et OH,
ou dans laquelle
R³ représente un hétérocycle de sept, huit, neuf ou dix chaînons, saturé ou partiellement saturé, bi- ou polycyclique, qui contient un atome d'azote et qui est relié par cet atome d'azote au radical de la molécule et qui peut contenir éventuellement un, deux ou trois autres atomes choisis dans le groupe comprenant un atome d'azote, de soufre et d'oxygène et qui peut être substitué éventuellement par un ou plusieurs radicaux choisis dans le groupe comprenant OH, (un atome d'halogène), un groupe alkyle en C₁ à C₆, alcanol en C₁ à C₆, alkyle en C₁ à C₄-O-alkyle en C₁ à C₃, aryle en C₆ à C₁₀, cycloalkyle en C₃ à C₁₀, un groupe hétéroaryle de cinq à dix chaînons, un hétérocycle de quatre à dix chaînons, un groupe alkylène en C₁ à C₂-hétéroaryle en C₅ à C₁₀ et alkylène en C₁ à C₂-hétérocycle en C₄ à C₁₀,
qui peut éventuellement être substitué à nouveau par un ou plusieurs radicaux choisis dans le groupe comprenant un groupe méthyle, éthyle, O-méthyle, Cl, F et OH,
et leurs sels, diastéréomères, énantiomères, racémates, hydrates ou solvates pharmaceutiquement acceptables.

4. Composés de formule 1 selon l'une des revendications 1 à 3, dans laquelle R¹ représente un groupe pyrrolidine ou azétidine et leurs sels, diastéréomères, énantiomères, racémates, hydrates ou solvates pharmaceutiquement acceptables.

5. Composés de formule 1 selon l'une des revendications 1 à 3, dans laquelle
R² représente un groupe pipérazine, et leurs sels, diastéréomères, énantiomères, racémates, hydrates ou solvates pharmaceutiquement acceptables.

6. Composés de formule 1 selon l'une des revendications 1 à 5, dans laquelle
R³ représente NHR^{3.1},
leurs sels, diastéréomères, énantiomères, racémates, hydrates ou solvates pharmaceutiquement acceptables.

7. Composés de formule 1 selon la revendication 6, dans laquelle
R^{3.2} représente un groupe alcanol en C₁ à C₆ ou cycloalkyle en C₃ à C₆ ramifié ou non ramifié, mono- ou multivalent et leurs sels, diastéréomères, énantiomères, racémates, hydrates ou solvates pharmaceutiquement acceptables.

8. Composés de formule 1 selon l'une des revendications 1 à 5, dans laquelle
R³ représente NHR^{3.1} ou OR^{3.1}
R^{3.1} représente un hétérocycle saturé ou non saturé, de cinq ou six chaînons, comportant 1 ou 2 hétéroatomes choisis indépendamment dans le groupe comprenant O,S et N,
qui comprend éventuellement au moins l'un des radicaux choisis dans le groupe comprenant
OH, un groupe méthyle, éthyle, alcanol en C₁ à C₄ ramifié ou non ramifié, phényle et cycloalkyle en C₃ à C₁₀.

9. Composés de formule 1 selon la revendication 8, dans laquelle
R^{3.1} est un groupe tétrahydrofuryle ou tétrahydropyranyle qui peut être substitué éventuellement par au moins l'un des radicaux choisis dans le groupe comprenant
OH, un groupe méthyle, éthyle, alcanol en C₁ à C₄ ramifié ou non ramifié, phényle et cycloalkyle en C₃ à C₁₀.

10. Composés de formule 1 selon l'une des revendications 1 à 5, dans laquelle
R³ représente un radical choisi dans le groupe comprenant un hétérocycle saturé de cinq ou six chaînons et un hétérocycle de huit, neuf ou dix chaînons bicyclique saturé ou partiellement saturé qui contient un atome d'azote et est relié par cet atome d'azote au radical de la molécule et qui peut contenir éventuellement un autre atome choisi dans le groupe comprenant un atome d'azote, de soufre et d'oxygène et qui peut être substitué éventuellement par un ou plusieurs radicaux choisis dans le groupe comprenant OH, un groupe méthyle, éthyle, alcanol en C₁ à C₄ ramifié ou non ramifié, phényle, cycloalkyle en C₃ à C₁₀, hétéroaryle de cinq à dix chaînons et un hétérocycle de quatre à dix chaînons,
et leurs sels, diastéréomères, énantiomères, racémates, hydrates ou solvates pharmaceutiquement acceptables.

11. Composés de formule 1 selon la revendication 10, dans laquelle
R³ représente un groupe pyrrolidine qui est relié par l'atome d'azote à la molécule restante et qui peut être substitué éventuellement par un ou plusieurs radicaux choisis dans le groupe comprenant OH, un groupe méthyle, éthyle et alcanol en C₁ à C₄ ramifié ou non ramifié,
et leurs sels, diastéréomères, énantiomères, racémates, hydrates ou solvates pharmaceutiquement acceptables

12. Composés de formule 2 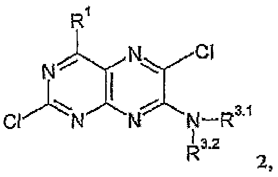 de formule 3 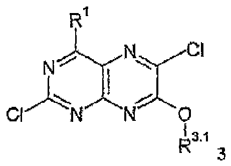 ou de formule 4 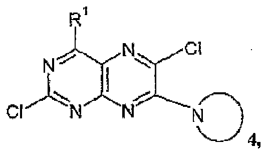 dans lesquelles R^{3.1} et R^{3.2} ont les significations définies dans la revendication 1,
dans lesquelles les R¹ ont les significations pyrrolidinyle, azétidinyle ou thiomorpholinyle et dans lesquelles 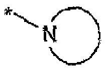 est choisi dans le groupe comprenant un hétérocycle monocyclique de quatre, cinq, six ou sept chaînons saturé ou partiellement saturé ou un hétérocycle bicyclique de sept à dix chaînons qui est relié par un atome d'azote au radical de la molécule et qui peut contenir éventuellement un ou deux autres atomes choisis parmi un atome d'azote, de soufre et d'oxygène et
qui peut être substitué éventuellement par un ou plusieurs radicaux choisis parmi OH, (un atome d'halogène), un groupe alkyle en C₁ à C₆, alcanol en C₁ à C₆, alkyle en C₁ à C₄-O-alkyle en C₁ à C₃, aryle en C₆ à C₁₀, cycloalkyle en C₃ à C₁₀, un groupe hétéroaryle de cinq à dix chaînons, un hétérocycle de quatre à dix chaînons, un groupe alkylène en C₁ à C₂-hétéroaryle en C₅ à C₁₀ et alkylène en C₁ à C₃-hétérocycle en C₄ à C₁₀,
qui peut éventuellement être substitué à nouveau par un ou plusieurs radicaux choisis dans le groupe comprenant un groupe méthyle, éthyle, O-méthyle, Cl, F et OH.

13. Composés selon l'une des revendications 1 à 11 comme médicament.

14. Utilisation de composés selon l'une des revendications 1 à 11, pour la production d'un médicament pour le traitement des troubles ou maladies des voies respiratoires ou gastro-intestinales ainsi que des maladies inflammatoires des articulations, de la peau ou des yeux, les cancers et les maladies du système nerveux périphérique ou central.

15. Utilisation de composés selon l'une des revendications 1 à 11, pour la production d'un médicament pour la prévention et le traitement des maladies des voies respiratoires ou pulmonaires qui s'accompagnent d'une production accrue de mucus, des inflammations et/ou des maladies obstructives des voies respiratoires.

16. Utilisation de composés selon l'une des revendications 1 à 11, pour la production d'un médicament pour le traitement des maladies inflammatoires et obstructives telles que la BPCO, la sinusite chronique, l'asthme, la maladie de Crohn, la reotocolite hémorragique.

17. Utilisation de composés selon l'une des revendications 1 à 11, pour la production d'un médicament pour la prévention et le traitement de maladies du système nerveux périphérique ou central telles que la dépression, la dépression bipolaire ou maniaque, les angoisses aiguës et chroniques, la schizophrénie, la maladie d'Alzheimer, la maladie de Parkinson, la sclérose en plaque aiguë et chronique ou les douleurs aiguës et chroniques et les lésions du cerveau provoquées par un AVC, une hypoxie ou un traumatisme crânien.

18. Utilisation de composés selon l'une des revendications 1 à 11, pour la production d'un médicament pour le traitement des cancers tels que les leucémies aiguës et chroniques, la leucémie lymphatique et la leucémie myéloïde aiguë, la leucémie lymphatique chronique et la leucémie myéloïde chronique, les maladies des organes lymphatiques, les lymphomes hodgkiniens et non hodgkiniens et les tumeurs osseuses telles que l'ostëosarcome et toutes les sortes de gliomes tels que l'oligodendrogliome et le glioblastome.
